(19) Europäisches Patentamt
European Patent Office
Office européen des brevets

(11) **EP 4 571 356 A1**

(12) **DEMANDE DE BREVET EUROPEEN**

(43) Date de publication:
**18.06.2025 Bulletin 2025/25**

(21) Numéro de dépôt: **24215535.6**

(22) Date de dépôt: **26.11.2024**

(51) Classification Internationale des Brevets (IPC):
**G01S 7/52** (2006.01)   **G01S 15/89** (2006.01)

(52) Classification Coopérative des Brevets (CPC):
**G01S 7/52036; G01S 7/52049; G01S 7/52052; G01S 15/8915; G01S 15/8977**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **13.12.2023 FR 2314128**

(71) Demandeurs:
• **SUPERSONIC IMAGINE**
**13290 Aix-en-Provence (FR)**
• **Centre National de la Recherche Scientifique**
**75016 Paris (FR)**
• **Ecole Supérieure de Physique et de Chimie Industrielles de la Ville de Paris**
**75005 Paris (FR)**
• **Université Paris Cité**
**75006 Paris (FR)**

(72) Inventeurs:
• **GIRAUDAT, Elsa**
**78830 BULLION (FR)**
• **BUREAU, Flavien**
**75005 PARIS (FR)**
• **LE BER, Arthur**
**76760 VIBEUF (FR)**
• **LAMBERT, William**
**13290 AIX EN PROVENCE (FR)**
• **ETAIX, Nicolas**
**13290 AIX EN PROVENCE (FR)**
• **FINK, Mathias**
**92190 MEUDON (FR)**
• **AUBRY, Alexandre**
**94200 IVRY SUR SEINE (FR)**

(74) Mandataire: **Plasseraud IP**
**104 Rue de Richelieu**
**CS92104**
**75080 Paris Cedex 02 (FR)**

(54) **PROCÉDÉ ET SYSTÈME DE CARACTÉRISATION ULTRASONORE D'UN MILIEU**

(57) Procédé de caractérisation ultrasonore d'un milieu comprenant des étapes de génération d'une série d'ondes ultrasonores incidentes, mesure d'une matrice de réflexion canonique $\mathbf{R}_{ui}(t)$ définie entre la base d'émission ($\mathbf{i}$) en entrée et une base de réception ($\mathbf{u}$) en sortie, détermination d'un ensemble de réponses R du milieu obtenues à partir de la matrice de réflexion canonique $\mathbf{R}_{ui}(t)$, à plusieurs fréquences $f$ et pour plusieurs points dans une région autour d'un point de référence, détermination d'une loi de correction fréquentielle $\Phi$ à partir des réponses du milieu aux différents points, la loi de correction fréquentielle étant adaptée au point de référence et étant déterminée aux fréquences $f$, détermination des réponses corrigées $R'$ du milieu par application de la loi de correction fréquentielle $\Phi$ aux réponses $R$ du milieu autour du point de référence et pour la pluralité des fréquences $f$.

FIG. 5

## Description

## DOMAINE TECHNIQUE

La présente divulgation concerne des procédés et systèmes de caractérisation par ultrasons d'un milieu hétérogène. Ces procédés et systèmes mettent en oeuvre un réseau de transducteurs placé en contact avec un milieu, pour émettre des ondes ultrasonores dans ce milieu et pour mesurer les ondes rétrodiffusées par les hétérogénéités de ce même milieu.

## ETAT DE L'ART

Dans le domaine de l'imagerie acoustique, on cherche à caractériser un milieu en le sondant de manière active avec des ondes ultrasonores. C'est notamment le principe de l'échographe en imagerie médicale.

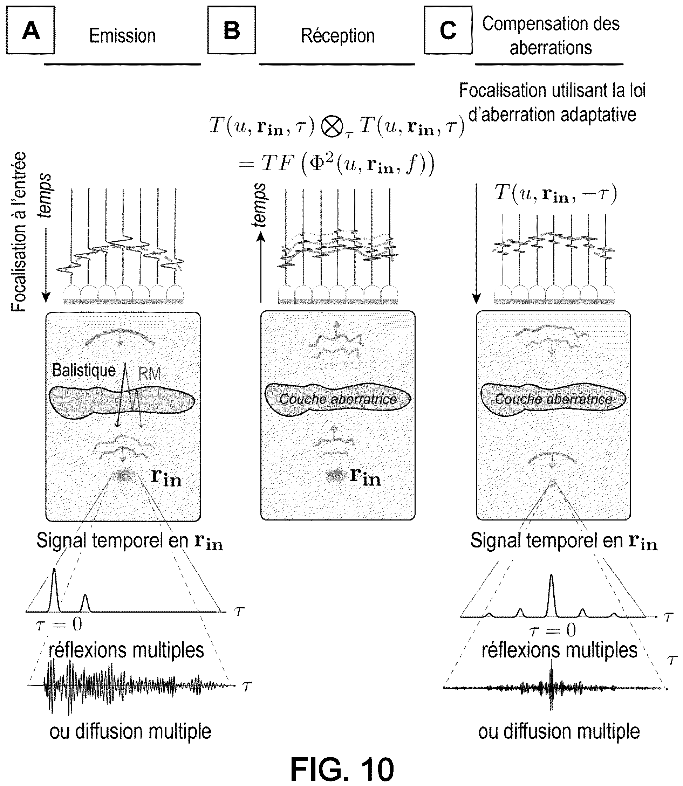

La **figure 10** illustre un processus conventionnel de focalisation dans un milieu pour produire une image échographique de ce milieu. Le milieu comprend dans cet exemple une couche aberratrice avec une vitesse du son différente de la vitesse du son observée dans les autres parties du milieu. Il en résulte une distorsion spatiale et une dispersion temporelle (réverbération) du front d'ondes acoustiques qui conduit à des aberrations transverses et axiales de l'image échographique résultante. Ces phénomènes conduisent à une dégradation de sa résolution et de son contraste ainsi qu'à l'apparition d'artefacts de réverbérations, particulièrement gênants lors d'un examen médical par exemple.

Sur le schéma de gauche de cette figure, le réseau de transducteurs, placé en vis-à-vis d'un milieu, permet d'insonifier et d'imager le milieu. La méthode conventionnelle consiste à insonifier le milieu à l'aide d'émissions focalisées par une technique dite de formation de voies (ou "beamforming" en langue anglaise). On applique aux signaux émis par chaque transducteur un jeu de retards appropriés $\tau$ basés sur un modèle de vitesse homogène $c_0$, afin de faire interférer constructivement les ondes produites par chaque transducteur au point focal visé de position spatiale $\mathbf{r}_{in}. = (x_{in}, z)$. En raison des limites physiques de la diffraction, les ultrasons émis au travers de l'ouverture de la sonde échographique sont concentrés dans une zone souvent nommée « tache focale ». En outre, les ondes traversant la couche aberratrice sont déformées et réfléchies plusieurs fois, ce qui provoque une pluralité d'échos d'ondes en direction du point focal pendant cette émission.

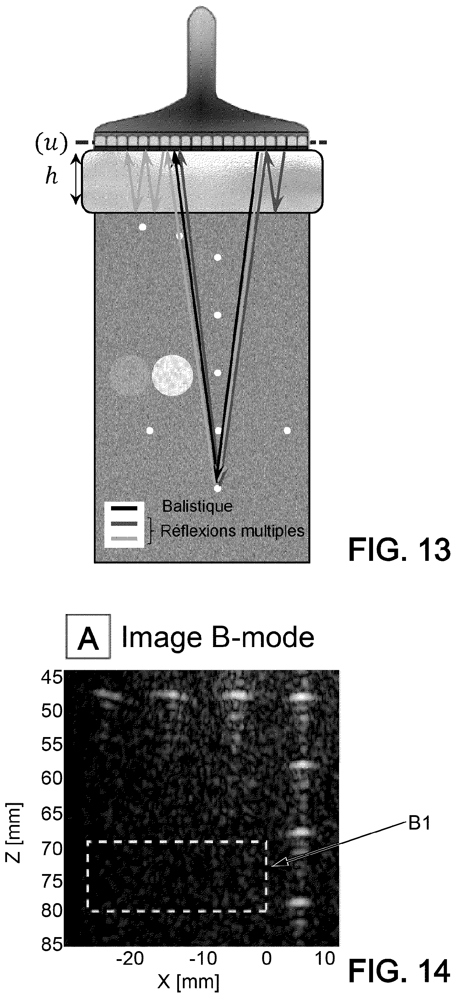

Sur le schéma au centre de cette figure, les ondes réfléchies au point focal sont retournées vers le réseau de transducteurs puis traversent à nouveau la couche aberratrice, ce qui déforme encore plus les ondes ultrasonores, et provoque une multiplication d'échos à cause des réflexions multiples. Chaque diffuseur du milieu donne ainsi lieu à plusieurs échos générant plusieurs impulsions temporelles parvenant à des temps différents sur chaque transducteur, d'où une importante dispersion temporelle des signaux ultrasonores. Un processus de formation de voie appliqué à ce type de signaux permet de construire une image échographique présentant une importante distorsion axiale : un même diffuseur apparaît à plusieurs profondeurs comme illustré par l'image échographique de la **figure 14.** De plus, la distribution hétérogène de vitesse du son dans les tissus traversés impacte la qualité de l'image construite.

Le schéma de droite de la **figure 10** explicite comment on pourrait, dans le cas d'un seul diffuseur, déconvoluer temporellement les signaux reçus par chaque transducteur puis les retourner temporellement, pour focaliser de manière optimale les ondes ultrasonores à la fois spatialement et temporellement sur le diffuseur en question. Cette technique de focalisation par retournement temporel reste limitée car elle nécessite un milieu ne comprenant que quelques diffuseurs. En échographie ultrasonore, le défi est tout autre puisque l'on a des milieux présentant une répartition aléatoire d'une multitude de diffuseurs sous-résolus générant un speckle ultrasonore.

Ainsi l'hypothèse habituelle d'un milieu homogène avec une vitesse du son $c_0$ constante de l'imagerie conventionnelle n'est souvent pas respectée. Les ondes sont alors réverbérées avec des multiples réflexions internes sur le parcours des ondes vers un point focal. Il en résulte une distorsion spatio-temporelle du front d'onde acoustique qui conduit à des aberrations significatives de l'image échographique, et donc à une dégradation de sa résolution et de son contraste. Ces aberrations peuvent être telles qu'elles compromettent la caractérisation ultrasonore.

Le document WO 2020/016250 propose une technique de correction des aberrations en imagerie ultrasonore basée sur une manipulation post-traitement de la matrice de réflexion du milieu. Cependant, le procédé décrit dans ce document ne considère que des signaux ultrasonores IQ fenêtrés temporellement autour du temps balistique attendu. Le déphasage appliqué sur ces signaux pour corriger les aberrations est donc équivalent à une application de retards temporels, ces retards devant être par ailleurs d'amplitude inférieure à la résolution temporelle des signaux ultrasonores. La technique du document WO 2020/016250 s'applique donc à des corrections d'aberration d'ordre relativement faible et sans dispersion temporelle. Elle ne peut donc pas être utilisée pour corriger des problèmes de réverbération ou de diffusion multiple qui nécessitent de déterminer des lois de retard différentes pour chaque composante fréquentielle du signal ultrasonore.

**RESUME**

La présente divulgation a pour objet de perfectionner les procédés de sondage par ultrasons connus, notamment afin de corriger des aberrations.

**La présente divulgation a pour objet, selon un premier aspect, un procédé de caractérisation ultrasonore** pour analyse médicale d'un milieu, le procédé comprenant les étapes de :

- génération d'une série d'ondes ultrasonores incidentes dans une zone dudit milieu, au moyen d'un réseau de transducteurs, ladite série d'ondes ultrasonores incidentes étant une base d'émission (**i**) ; et
- mesure d'une matrice de réflexion canonique $\mathbf{R}_{ui}(t)$ définie entre la base d'émission (**i**) en entrée et une base de réception (**u**) en sortie, les coefficients de cette matrice de réflexion canonique correspondant aux signaux reçus par les transducteurs et induits par les ondes ultrasonores réfléchies dans le milieu.

Le procédé comprend en outre les étapes de :

- détermination (S 130) d'un ensemble de réponses $R$ du milieu qui sont obtenues par un processus de focalisation pour plusieurs fréquences $f$ des signaux reçus des ondes ultrasonores réfléchies et pour plusieurs points de position spatiale $\mathbf{r} = (x, z)$ d'une région autour d'un point de référence de position spatiale $\mathbf{r}_p = (x_p, z_p)$ à partir de la matrice de réflexion canonique $\mathbf{R}_{ui}(t)$ pour un modèle de vitesse du son $c_0$,
- détermination (S 140) d'une loi de correction fréquentielle $\Phi$ par moyenne ou corrélation des réponses du milieu aux différents points de position spatiale $(x,z)$ autour du point de référence, la loi de correction fréquentielle étant adaptée au point de référence et étant déterminée aux fréquences $f$,
- détermination (S 180) des réponses corrigées $R'$ du milieu par application de la loi de correction fréquentielle $\Phi$ aux réponses $R$ du milieu autour du point de référence et pour la pluralité des fréquences $f$.

**Grâce à ces dispositions,** le procédé permet avantageusement de sonder localement le milieu pour obtenir une estimation locale d'une loi de correction fréquentielle adaptée pour corriger le processus de focalisation ultrasonore. Cette correction permet de réduire ou supprimer des aberrations par exemple dues à des réflexions multiples des ondes générées par une ou plusieurs zones aberratrices dans le milieu.

Ces estimations sont effectuées par calcul à partir de mesures effectuées et enregistrées dans une matrice de réflexion canonique. Ces estimations peuvent ainsi être calculées indépendamment de la phase d'acquisition des mesures, en particulier en modifiant divers paramètres de calcul, ce qui permet de procéder à diverses analyses de caractérisation ultrasonore soit en temps réel, soit a posteriori.

Ces calculs de correction bénéficient d'informations locales extraites autour d'un point de référence et d'informations locales fréquentielles du milieu.

Le procédé peut être utilisé en imagerie médicale ou vétérinaire ainsi qu'à tout domaine d'imagerie par ultrasons.

Selon divers modes de réalisation du procédé, on peut éventuellement avoir recours en outre à l'une et/ou à l'autre des dispositions suivantes.

Selon une variante, le procédé comprend en outre une étape de :

- détermination (S190) d'une intensité $I_c$ d'un point d'image échographique correspondant au point de référence de position spatiale $\mathbf{r}_p$ par combinaison des réponses corrigées à plusieurs fréquences $f$ de ce point de référence.

Selon une variante :

- la détermination (S130) de l'ensemble des réponses $R$ du milieu comprend la détermination de réponses qui sont obtenues par le processus de focalisation entre un premier point de position spatiale $\mathbf{r}_{in} = (x_{in}, z)$ correspondant à un transducteur virtuel d'entrée et un deuxième point de position spatiale $\mathbf{r}_{out} = (x_{out}, z)$ correspondant à un transducteur virtuel de sortie, le premier point et le deuxième point étant identiques ($\mathbf{r}_{in} = \mathbf{r}_{out}$), et
- l'ensemble des réponses $R$ étant enregistrées dans une matrice de réflexion confocale $\mathbf{R}$ dont les coefficients s'écrivent selon $\mathbf{R} = [R(x, z, f)]$,
- la détermination (S 140) de la loi de correction fréquentielle $\Phi$ est effectuée par corrélation des réponses du milieu aux différents points de position spatiale $(x, z)$ autour du point de référence, et dont les coefficients s'écrivent $\Phi = [\phi(f, \mathbf{r}_p)]$
- la détermination (S180) des réponses corrigées $R'$ autour du point de référence, par application de la loi de correction fréquentielle à chaque fréquence $f$, en effectuant le produit terme à terme entre la matrice de réflexion confocale $\mathbf{R}$ et le conjugué en phase de la loi de correction fréquentielle $\Phi$, c'est-à-dire par :

$$\mathbf{R}' = \mathbf{R} \circ \mathbf{\Phi}^*$$

où

l'ensemble des réponses corrigées *R'* sont enregistrées dans une matrice de réflexion confocale corrigée **R'** dont les coefficients s'écrivent selon **R'** = [*R'(x, z, f)*]
le symbole ∘ est le produit d'Hadamard, tel que :

$$R'(x, z, f) = R(x, z, f)\phi^*(f, \mathbf{r_p}).$$

Selon une variante, le procédé comprend en outre une étape de :

- détermination (S190) d'une intensité $I_c$ d'un point d'image échographique de position spatiale *(x, z)* par combinaison des réponses corrigées *R'* de ce point à plusieurs fréquences *f*, c'est-à-dire par :

$$I_c(x, z) = \left| \sum_f R'(x, z, f) \right|^2$$

Selon une variante :

la détermination d'une loi de correction fréquentielle (S140) comprend

la construction d'une matrice de corrélation (S141) **C** à partir de la matrice de réflexion confocale **R**(z,f), et une analyse (S142) de la matrice de corrélation C pour déterminer la loi de correction fréquentielle Φ.

Selon une variante :

la matrice de corrélation **C** est déterminée dans le domaine fréquentiel, par :

$$C(f, f') = \sum_{x,z} R(x, z, f)\ R^*(x, z, f')$$

où

**R** est la matrice de réflexion confocale,
*x, z* sont les coordonnées des points de la région autour du point de référence,
* est l'opérateur de conjugaison.

Selon une variante :

la matrice de corrélation **C** est déterminée dans une base des points d'image de position spatiale *(x, z)*, par :

$$C(\{x, z\}, \{x', z'\}) = \sum_f R(x, z, f)\ R^*(x', z', f)$$

où

**R** est la matrice de réflexion confocale,
*x, z* sont les coordonnées des points de l'image dans la région autour du point de référence,
* est l'opérateur de conjugaison.

Selon une variante :
l'analyse (S 142) de la matrice de corrélation **C** est une décomposition en valeurs propres de la matrice de corrélation C et la loi de correction fréquentielle Φ est le premier vecteur propre **U₁** de la matrice de corrélation **C.**

Selon une variante :
l'analyse (S 142) de la matrice de corrélation **C** est la résolution d'une équation faisant intervenir la matrice de corrélation **C** et la loi de correction fréquentielle Φ, cette résolution d'équation correspondant à un retournement temporel itératif ou un retournement de phase itératif.

Selon une variante :
la détermination (S140) de la loi de correction fréquentielle est effectuée par un algorithme d'optimisation maximisant l'intensité confocale d'une image échographique dans la région autour du point de référence.

Selon une variante :

les étapes (S140, S180) du traitement de correction sont itérées plusieurs fois, et
dans lequel à chaque itération, on utilise la matrice de réflexion confocale corrigée **R'**(*z,f*) obtenue à l'itération précédente à la place de la matrice de réflexion focalisée **R**(*z,f*).

Selon une variante :
à chaque itération, la taille de la région autour du point de référence de position spatiale **r**$_p$ est réduite.

Selon une variante :
la détermination (S130) de la matrice de réflexion confocale **R**(*z,f*) comprend une compensation de l'atténuation en temps des signaux.

Selon une variante :

- la détermination (S130) de l'ensemble des réponses *R* du milieu comprend la détermination de réponses qui sont obtenues par le processus de focalisation entre un premier point de position spatiale **r**$_{in}$ = *(x$_{in}$, z)* correspondant à un transducteur virtuel d'entrée et un deuxième point de position spatiale **r**$_{out}$ = *(x$_{out}$, z)* correspondant à un transducteur virtuel de sortie, le premier point et le deuxième point étant situés dans la région à la même profondeur *z,* les positions transverses *x$_{in}$* et *x$_{out}$* des premier et deuxième points formant une base focalisée (**x**) à chaque profondeur *z,* et l'ensemble des réponses *R* étant enregistrés dans une matrice de réflexion focalisée **R**$_{xx}$(*z,f*) dont les coefficients s'écrivent selon **R**$_{xx}$(*z, f*) = [*R*(*x$_{in}$, x$_{out}$, z, f*)],
- la détermination (S140) de la loi de correction fréquentielle Φ comprend des sous-étapes effectuées à chaque profondeur *z* et chaque fréquence *f*, de :

    - détermination (S150) d'une matrice de réflexion duale **R**$_c$(*z,f*) par projection aller de la matrice de réflexion focalisée **R**$_{xx}$(*z,f*) vers une base de correction (**c**),
    - calcul (S160) de la loi de correction fréquentielle Φ à partir de la matrice de réflexion duale **R**$_c$(*z,f*), ladite loi de correction fréquentielle étant déterminée sur la base de correction (**c**), Φ = [$\phi$(*c, f,* **r**$_p$)], de sorte que ladite loi de correction fréquentielle Φ est une loi de correction spatio-fréquentielle,

    - détermination (S170) d'une matrice de réflexion duale corrigée $\mathbf{R}'_c$ autour du point de référence et dont les coefficients s'écrivent selon $\mathbf{R}'_c(z, f) = [R'_c(x, c, f, z)]$, déterminée en effectuant le produit terme à terme entre la matrice de réflexion duale **R**$_c$(*z, f*) et le conjugué en phase de la loi de correction fréquentielle Φ, c'est-à-dire par :

$$\mathbf{R}'_c = \mathbf{R}_c \circ \boldsymbol{\Phi}^*$$

    où

    le symbole * désigne une opération de conjugaison de phase
    le symbole ∘ est le produit d'Hadamard, tel que :

$$R'_c(x, c, f, z) = R_c(x, c, f, z)\Phi^*(x, c, f, z)$$

- la détermination (S180) des réponses corrigées *R'* du milieu autour du point de référence comprend la détermination d'une matrice de réflexion focalisée corrigée $\mathbf{R}'_{xx}(z, f)$ par projection retour de la matrice de réflexion duale corrigée **R'**$_c$(*z,f*) vers la base focalisée (**x**).

Selon une variante, le procédé comprend en outre une étape de :

- détermination (S190) d'une intensité *I$_c$* d'un point d'image échographique de position spatiale (*x, z*), par combinaison des coefficients diagonaux de la matrice de réflexion focalisée corrigée **R'**$_{xx}$(*z,f*) de ce point, à plusieurs fréquences *f*, c'est-à-dire :

$$I_c(x,z) = \left| \sum_f R'_{xx}(x,x,z,f) \right|^2$$

Selon une variante :

la projection aller (150) est effectuée par un produit matriciel entre une matrice de passage et la matrice de réflexion focalisée $\mathbf{R}_{xx}(z,f)$, c'est-à-dire :

$$\mathbf{R}_c(z,f) = \mathbf{P}(z,f) \times \mathbf{R}_{xx}(z,f)$$

où : $\mathbf{P}(z,f) = [P(c, x, z, f)]$ est la matrice de passage à chaque fréquence $f$ entre la base focalisée ($\mathbf{x}$) à la profondeur $z$ et la base de correction ($\mathbf{c}$).

Selon une variante :
la base de correction ($\mathbf{c}$) est une base de correction en entrée ou une base de correction en sortie.
Selon une variante :
Le calcul de la loi de correction fréquentielle (S 160) comprend :

la construction d'une matrice de corrélation (S161) $\mathbf{C}$ à partir de la matrice de réflexion duale $\mathbf{R}_c(z,f)$, et
une analyse (S162) de la matrice de corrélation C pour déterminer la loi de correction fréquentielle $\Phi$.

Selon une variante :

la matrice de corrélation $\mathbf{C}$ est déterminée dans la base de correction ($\mathbf{c}$) et dans le domaine fréquentiel, par :

$$C(\{c,f\},\{c',f'\}) =$$
$$\sum_{x,z} R_c(x,c,z,f) R^*_{ref}(x,c,z,f)\ R^*_c(x,c',z,f')\ R_{ref}(x,c',z,f')$$

où

$\mathbf{R}_c$ est la matrice de réflexion duale,
$\mathbf{R}_{ref}$ est une matrice de réflexion modèle d'un milieu modèle dans lequel la vitesse du son est $c_0$ vitesse du son attendue du milieu et dans lequel un réflecteur plan est positionné à la profondeur $z$,
$x, z$ sont les coordonnées des points de la région autour du point $\mathbf{r}_p$,
* est l'opérateur de conjugaison.

Selon une variante :

la matrice de corrélation $\mathbf{C}$ est déterminée dans une base des points d'image de position spatiale ($x, z$), par :

$$C_{rr}(\{x,z\},\{x',z'\}) =$$
$$\sum_{c,f} R_c(x,c,z,f) R^*_{ref}(x,c,z,f)\ R^*_c(x',c,z',f)\ R_{ref}(x',c,z',f)$$

où

$\mathbf{R}_c$ est la matrice de réflexion duale,
$\mathbf{R}_{ref}$ est une matrice de réflexion modèle d'un milieu modèle dans lequel la vitesse du son est $c_0$ vitesse du son attendue du milieu et dans lequel un réflecteur plan est positionné à la profondeur $z$,
$x, z$ sont les coordonnées des points de l'image dans la région autour du point $\mathbf{r}_{p\cdot}$,
* est l'opérateur de conjugaison.

Selon une variante :
l'analyse (S162) de la matrice de corrélation $\mathbf{C}$ est une décomposition en valeurs propres de la matrice de corrélation C et la loi de correction fréquentielle $\Phi$ est le premier vecteur propre $\mathbf{U_1}$ de la matrice de corrélation $\mathbf{C}.$
Selon une variante :

## EP 4 571 356 A1

l'analyse (S162) de la matrice de corrélation **C** est la résolution d'une équation faisant intervenir la matrice de corrélation **C** et la loi de correction fréquentielle $\Phi$, cette résolution d'équation correspondant à un retournement temporel itératif ou un retournement de phase itératif.

Selon une variante :

Le calcul de la loi de correction fréquentielle (S160) est effectué par un algorithme d'optimisation maximisant l'intensité confocale d'une image échographique dans la région autour du point de référence.

Selon une variante :

les étapes (S140, S180) du traitement de correction sont itérées plusieurs fois, et

dans lequel à chaque itération, la projection aller (S150) utilise la matrice de réflexion focalisée corrigée $\mathbf{R'}_{xx}(z,f)$ obtenue durant la projection retour (S180) de l'itération précédente à la place de la matrice de réflexion focalisée $\mathbf{R}_{xx}(z,f)$.

Selon une variante :

à chaque itération de la projection aller (S 150), on alterne entre une projection aller dans une base de correction en entrée et une base de correction en sortie.

Selon une variante :

à chaque itération, la base de correction (c) de la projection aller (S150) est différente.

Selon une variante :

à chaque itération, la taille de la région autour du point de référence de position spatiale $\mathbf{r}_p$ est réduite

Selon une variante :

la détermination (S130) de la matrice de réflexion focalisée $\mathbf{R}_{xx}(z,f)$ comprend une compensation de l'atténuation en temps des signaux.

**La présente divulgation concerne, selon un deuxième aspect, un système de caractérisation ultrasonore** pour analyse d'un milieu, par exemple dans le contexte médical, et configuré pour la mise en oeuvre de procédés tels que décrits précédemment. Le système selon le deuxième aspect comprend :

- un réseau de transducteurs adaptés pour générer une série d'ondes ultrasonores incidentes dans une zone du milieu, et pour mesurer en fonction du temps les ondes ultrasonores rétrodiffusées par ladite zone ; et
- une unité de calcul associée au réseau de transducteurs et adaptée pour mettre en oeuvre le procédé selon le premier aspect.

## BREVE DESCRIPTION DES FIGURES

D'autres avantages et caractéristiques de la technique présentée ci-dessus apparaîtront à la lecture de la description détaillée ci-dessous, présentée de manière non limitative à des fins d'illustration, faite par référence aux figures dans lesquelles :

Les **figures 1(a) à 1(h)** illustrent des séquences d'émission/réception utilisées pour l'imagerie et la caractérisation ultrasonore d'un milieu ;

La **figure 2** illustre l'imagerie confocale usuelle et l'imagerie matricielle avec la synthèse d'une matrice de réflexion focalisée contenant les réponses entre des transducteurs virtuels distincts synthétisés à chaque profondeur par formation de voies ;

La **figure 3** illustre un exemple de système de caractérisation ultrasonore pour la mise en oeuvre du procédé selon la présente divulgation ;

La **figure 4** présente les définitions utilisées dans le procédé selon la présente divulgation ;

La **figure 5** est un diagramme du procédé de caractérisation ultrasonore selon la présente divulgation, dans lequel on détermine une loi de correction fréquentielle à partir de réponses en plusieurs points du milieu et à plusieurs fréquences et on corrige les réponses du milieu en appliquant cette loi de correction fréquentielle ;

La **figure 6** est un diagramme d'un mode de réalisation de l'étape de détermination d'une loi de correction fréquentielle du procédé de la figure 5, par projection de la matrice de réflexion focalisée dans une base de correction ;

La **figure 7** illustre un calcul particulier de loi de correction fréquentielle de la figure 6, par construction et analyse d'une matrice de corrélation ;

La **figure 8** illustre un calcul particulier de loi de correction fréquentielle de la figure 6 par optimisation d'image échographique locale ;

La **figure 9** illustre un diagramme d'un mode de réalisation de l'étape de détermination d'une loi de correction fréquentielle du procédé de la figure 5, par construction et analyse d'une matrice de corrélation, dans un cas de matrice de réflexion confocale ;

La **figure 10** illustre le problème d'une couche aberratrice en imagerie ultrasonore ;

La **figure 11** illustre le premier mode de réalisation du procédé selon la présente divulgation en version matricielle qui résout ce problème des réverbérations, généralisant au cas « speckle » le procédé décrit dans la figure 10 dans le cas d'un diffuseur isolé ;

La **figure 12** illustre le deuxième mode de réalisation du procédé selon la présente divulgation en version confocale qui résout ce problème des réverbérations ;

La **figure 13** illustre un milieu expérimental de type fantôme ultrasonore sur lequel le procédé de la présente divulgation est testé ;

La **figure 14** montre une image échographique d'une zone d'intérêt du milieu de la figure 13, image échographique présentant des artefacts de réverbération particulièrement visibles notamment au niveau des diffuseurs échogènes du milieu expérimental calibré ;

La **figure 15** illustre une loi de correction fréquentielle exprimée dans le domaine temporel et obtenue dans une région B1 de l'image de la figure 14 ;

La **figure 16** montre une image échographique sans correction en figure 16(a) et une image obtenue avec la correction du procédé selon la présente divulgation en figure 16(b) ;

La **figure 17** montre la même image échographique que la figure 14 avec trois régions sélectionnées pour appliquer le procédé de manière locale selon la présente divulgation ;

La **figure 18** illustre les lois de correction spatio-fréquentielle obtenues dans les régions C1, C2 et C3 de l'image de la figure 17 ;

La **figure 19** montre une image échographique sans correction en figure 19(a), une image échographique obtenue avec correction globale en figure 19(b), et une image échographique obtenue avec correction dans les trois régions C1, C2 et C3 en figure 19(c).

Dans les différents modes de réalisation décrits par référence aux figures, des éléments semblables ou identiques portent sauf stipulation contraire les mêmes références.

## DESCRIPTION DETAILLEE

Dans la description détaillée qui suit, seuls certains modes de réalisation sont décrits en détail pour assurer la clarté de l'exposé mais ces exemples ne visent pas à limiter la portée générale des principes ressortant de la présente divulgation.

Les différents modes de réalisation et aspects décrits dans la présente divulgation peuvent être combinés ou simplifiés de multiples manières. En particulier, les étapes des différents procédés peuvent être répétées, interverties, et/ou exécutées en parallèle, sauf précision contraire.

La présente divulgation concerne des procédés et systèmes de caractérisation ultrasonore d'un milieu, et s'applique notamment à l'imagerie médicale de tissus vivants ou non. Le milieu est par exemple un milieu hétérogène que l'on cherche à caractériser pour par exemple identifier et/ou caractériser les hétérogénéités, fournir des informations précises sur le milieu étudié, déceler des zones et/ou tissu non sains ou endommagés. A titre d'exemple, ces données sont très utiles pour des applications médicales telles qu'identification de lésions dans zone mammaire, tissus musculaires endommagés, ou dégradation des tissus du foie. Ces techniques de caractérisation sont notoirement non invasives pour le milieu, qui est avantageusement préservé en particulier dans sa nature et son intégrité.

### Approche usuelle de l'imagerie ultrasonore

Dans le domaine de l'imagerie par ultrasons, on cherche souvent à construire une image de la réflectivité d'un milieu à partir des échos rétrodiffusés par des hétérogénéités du milieu. C'est le principe de l'échographe utilisé en imagerie médicale, qui permet notamment de visualiser l'anatomie interne d'un objet, d'un individu ou d'un animal. A des fins de simplification, pour permettre la construction d'une image échographique, le milieu est considéré comme homogène, avec une vitesse de propagation du son $c_0$ constante.

Les méthodes d'échographie conventionnelle utilisent généralement un réseau de transducteurs piézo-électriques qui peuvent émettre et/ou recevoir des signaux ultrasonores de manière indépendante ou quasi indépendante, chaque transducteur étant à une position u dans la barrette supportant ledit réseau. Le réseau de transducteurs, placé en vis-à-vis d'un milieu, permet d'insonifier et de construire une image représentative du milieu de différentes manières. Une méthode conventionnelle consiste à insonifier le milieu à l'aide d'émissions focalisées par une technique dite de formation de voies (ou "beamforming" en langue anglaise). Cette méthode consiste à appliquer aux signaux émis par chaque transducteur un jeu de retards appropriés $\tau(u_{in}, x_{in}, z, c_0)$ basés sur un modèle de vitesse homogène $c_0$, afin de faire interférer constructivement les ondelettes produites par chaque transducteur au point focal visé de position spatiale $(x_{in}, z)$. En raison des limites physiques de la diffraction, les ultrasons sont émis au travers de l'ouverture de la sonde échographique, concentrés dans une zone souvent nommée « tache focale », de largeur latérale $\delta x$.

Afin de permettre de construire ensuite éventuellement une image échographique illustrant les caractéristiques du milieu étudié, une étape de focalisation numérique est également effectuée en réception. Les échos captés par les transducteurs du réseau, sont remis en phase en les décalant temporellement. Les délais $\tau(u_{out}, x_{out}, z, c_0)$ sont identiques à ceux appliqués à l'émission, la variable $u_{out}$ désignant la position de chaque transducteur. Dans la phase d'émission, tous les signaux interfèrent au point de position $(x_{in}, z)$ au temps balistique $t = z/c_0$ si le modèle de vitesse $c_0$ utilisé correspond à la réalité du milieu étudié. En réception, les signaux provenant de ce même point $(x_{out} = x_{in})$ interfèrent par sommation des signaux au temps d'écho $t = 2z/c_0$. Cette sommation permet d'obtenir le résultat final de la focalisation en réception. Cette méthode confocale à double focalisation à l'émission et à la réception permet d'imager directement la réflectivité du milieu avec une résolution latérale $\delta x$ et un bon contraste. Toutefois, cette méthode est chronophage car elle nécessite de focaliser physiquement à l'émission en chacun des points du milieu ou au moins à une profondeur donnée, sur chacune des lignes de l'image qui sera construite représentative du milieu.

**Approche matricielle de l'imagerie ultrasonore**

Une approche matricielle de l'imagerie ultrasonore a été développée ces dernières années par la demanderesse. Cette approche repose sur la construction d'une matrice de réflexion canonique du milieu étudié. Cette matrice de réflexion canonique est déterminée par expérience ou par calculs ou simulation numérique, reproduisant une expérience.

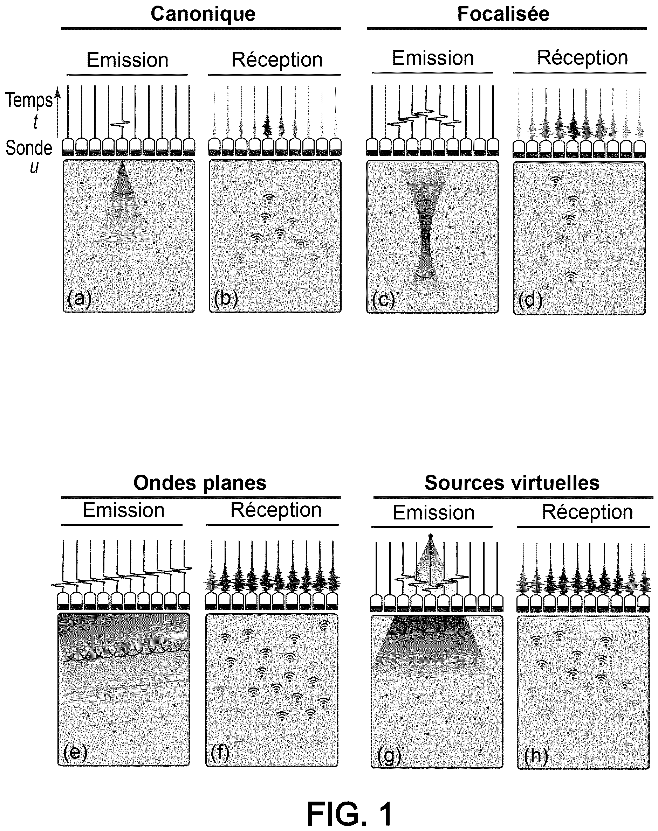

Une première proposition permettant de créer cette matrice de réflexion canonique est d'émettre successivement une impulsion ultrasonore depuis chaque transducteur du réseau dont la position est repérée par la coordonnée $u_{in}$, comme schématisé en **figure 1(a).** Cela donne lieu à une onde incidente cylindrique (ou sphérique) divergente. Cette onde est réfléchie par les diffuseurs du milieu et le champ rétrodiffusé est mesuré par chacun des transducteurs en fonction du temps comme représenté en **figure 1(b).** En répétant cette opération avec chaque transducteur utilisé successivement comme source, on détermine la matrice de réflexion canonique $\mathbf{R}_{uu}(t) = [R(u_{out}, u_{in}, t)]$ exprimée dans la base des transducteurs, composée de l'ensemble des réponses impulsionnelles $R(u_{out}, u_{in}, t)$ entre chaque transducteur. Cette matrice est alors riche de quantité d'informations décrivant le milieu étudié. Mais, la méthode suppose toutefois que le milieu reste fixe pendant toute la durée des mesures, ce qui est difficile en particulier dans le cas d'examens médicaux pratiqués sur des patients vivants. En outre, les signaux enregistrés possèdent un mauvais rapport signal à bruit car le milieu est insonifié par un seul transducteur.

Une deuxième alternative pour construire cette matrice de réflexion canonique est d'insonifier le milieu avec des ondes focalisées, successivement en une multitude de points du milieu, comme cela est généralement dans les échographes standards. La **figure 1(c)** illustre cette illumination focalisée à l'émission. Une loi de retard est appliquée sur chaque signal à l'émission pour cette focalisation. La réception illustrée en **figure 1(d)** est mesurés par tous les capteurs de de positions $u_{out}$ pour chaque illumination focalisée, et les réponses forment la matrice de réflexion canonique $\mathbf{R}(u_{out}, \mathbf{r}_{in}, t)$. Cependant, cette méthode de focalisation en de multiples points est encore trop lente.

Une troisième manière de construire cette matrice de réflexion canonique consiste à insonifier le milieu avec une série d'ondes planes. Cette méthode permet de s'affranchir de la plupart des problèmes inhérents à la méthode présentée précédemment. La **figure 1(e)** illustre le principe de cette illumination en ondes planes. Une loi de retard $\tau'$ est appliquée sur chaque signal à l'émission pour la formation d'un front d'onde incliné d'un angle $\theta_{in}$ par rapport au réseau de transducteurs. A la réception, comme illustré en **figure 1(f),** le champ rétrodiffusé par le milieu, $\mathbf{R}(u_{out}, \theta_{in}, t)$, est mesuré par tous les capteurs de positions $u_{out}$ pour chaque onde plane incidente $\theta_{in}$. L'ensemble de ces réponses forme une matrice de réflexion canonique $\mathbf{R}_{u\theta}(t) = [R(u_{out}, \theta_{in}, t)]$. La méthode de double focalisation décrite précédemment peut être réalisée numériquement en décalant temporellement les signaux mesurés avant de les sommer de manière cohérente. Cette méthode a donné naissance à l'imagerie ultrarapide, et à l'élastographie, et elle est par exemple décrite dans le document :

« Cohérent plane-wave compounding for very high frame rate ultrasonography and transient elastography », G. Montaldo et al. (IEEE Trans. Ultrason., Ferroelect. Freq. Control 56 489-506, 2009).

Une troisième alternative pour créer cette matrice de réflexion canonique consiste à insonifier le milieu avec une base d'ondes divergentes, comme représenté en **figure 1(g)** et **figure 1(h),** ce qui permet d'illuminer le champ acoustique de manière plus large que via l'utilisation des ondes planes. Cette technique utilisée notamment en imagerie de super-résolution, est explicitée dans le document :

« Ultrafast imaging of the heart using Circular Wave Synthetic Imaging with Phased Arrays», Couade et Al., IEEE International Ultrasonics Symposium (2009).

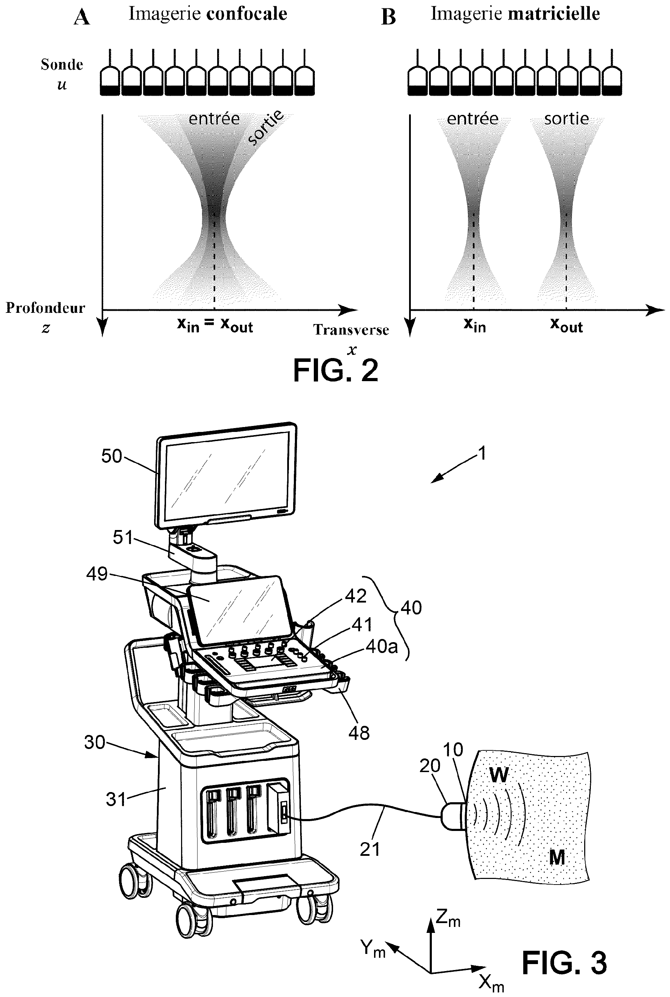

L'imagerie conventionnelle consiste donc en une double focalisation à l'émission et à la réception au même point focal pour chaque pixel de l'image $(x_{in} = x_{out})$, comme représenté en **figure 2(a).**

L'imagerie matricielle consiste quant à elle à dissocier les points focaux à l'émission et à la réception, pour un même temps d'écho $t$, comme représenté en **figure 2(b).** Ainsi, une matrice de réflexion focalisée $\mathbf{R}_{xx}(\tau) = [R(x_{in}, x_{out}, z, \tau)]$ est déterminée, cette matrice de réflexion focalisée étant composée des réponses entre des transducteurs virtuels d'entrée et des transducteurs virtuels de sortie de positions spatiales respectives $\mathbf{r_{in}} = (x_{in},\ z)$ et $\mathbf{r_{out}} = (x_{out},\ z)$ correspondant à des taches focales synthétisées en entrée et en sortie à des positions spatiales différentes, et dont la profondeur z attendue est dictée par le temps d'écho $t$ : $z=c_0t/2$. $\tau$ représente ici le temps dans la base focalisée tel que $\tau = t - 2z/c_0$. L'origine de ce temps $\tau$ correspond à l'instant où la source virtuelle émet l'impulsion ultrasonore. La matrice de réflexion focalisée est obtenue par focalisation (par exemple par des formations de voies) à partir de la matrice de réflexion canonique $\mathbf{R}_{ui}(t)$. Cette matrice permet d'accéder à d'avantage d'informations caractéristiques du milieu étudié que les informations obtenues via la méthode utilisée en mode image confocale de l'imagerie conventionnelle. Cette matrice de réflexion focalisée peut être synthétisée dans le domaine temporel par des lois de retard (formations de voies) ou dans le domaine fréquentiel en appliquant des déphasages appropriés.

La matrice de réflexion focalisée a été décrite en particulier dans le document :

« Reflection Matrix Approach for Quantitative Imaging of ScatteringMedia », William Lambert, et al., Phys. Rev. X 10, 021048, (2020),
puis dans le document
« Ultrasound Matrix Imaging - Part I: The Focused Reflection Matrix, the F-Factor and the Role of Multiple Scattering », William Lambert, et al., IEEE Trans. Med. Imag. 41, 3907-3920, (2022) .

Dans ces publications, la matrice de réflexion focalisée $\mathbf{R_{xx}}(z, \tau)$ a été considérée entre des transducteurs virtuels au temps balistique $\tau = 0$ ($t=2z/c_0$) Alors que les coefficients diagonaux ($x_{out} = x_{in}$) de la matrice $\mathbf{R_{xx}}(z, \tau=0)$ permettent de construire l'image confocale synthétique à la profondeur z, ses coefficients hors-diagonale nous renseignent sur les potentiels effets d'aberration et de diffusion multiple susceptibles d'altérer la qualité de cette même image.

Dans la publication de demande de brevet WO2020/016250, la matrice de réflexion focalisée a été considérée au temps balistique et étudiée pour notamment corriger les problèmes d'aberrations. Toutefois, la correction appliquée correspond à l'applications de retards temporels, ces retards devant être par ailleurs d'amplitude inférieur à la résolution temporelle des signaux ultrasonores. La technique du document WO 2020/016250 ne s'applique donc qu'à des corrections d'aberration d'ordre relativement faibles et sans dispersion temporelle. Il ne peut donc pas être utilisé pour corriger des problèmes de réverbération ou de diffusion multiple qui nécessitent de déterminer des lois de retard différentes pour chaque composante fréquentielle du signal ultrasonore

## Système de caractérisation ultrasonore

La **figure 3** illustre un exemple d'un système 1 d'imagerie ultrasonore pour la mise en oeuvre de procédés d'imagerie ultrasonore d'un milieu tel qu'un milieu hétérogène M, selon la présente divulgation. Ce système et procédé permettent la formation d'une image échographique par ultrasons d'au moins une partie (zone d'intérêt ou champ de vision) du milieu.

Le système 1 comprend :

- un dispositif de sondage 20 ou sonde 20,
- une unité de calcul 30 pour calculer une image à partir des signaux reçus de la sonde20,
- un panneau de contrôle 40 relié à l'unité de calcul 30, ce panneau de contrôle comprenant par exemple des boutons 41 et un pavé tactile 42,
- un dispositif d'affichage 50 pour visualiser une image et divers éléments ou mesures.

La sonde 20 est reliée à l'unité de calcul 30 via un câble 21 ou via une connexion sans fil, et est capable d'émettre des ondes ultrasonores W dans le milieu M et de recevoir des ondes ultrasonores W depuis le milieu M, lesdites ondes ultrasonores étant résultantes de réflexions des ondes ultrasonores émises sur des particules diffusantes ou diffuseurs à l'intérieur du milieu.

La sonde 20 peut comprendre un réseau 10 comprenant une pluralité de transducteurs 11. Le réseau 10 est par exemple un réseau linéaire ou courbé ou bidimensionnel ou matriciel. Les transducteurs 11 sont capables de convertir un signal électrique en une vibration et réciproquement. Les transducteurs 11 sont par exemple des transducteurs piézoélectriques ultrasonores pouvant se présenter sous la forme d'une barrette rigide mise en contact directement ou indirectement avec une surface externe du milieu M pour être couplé au milieu et pour faire vibrer et émettre et recevoir des ondes ultrasonores W. Le réseau 10 de transducteurs 11 de la sonde 20 est alors associé à l'unité de calcul 30. Le réseau 10 de transducteurs peut comprendre une centaine ou plus de transducteurs 11.

L'unité de calcul 30 peut comprendre un boitier 31 incluant des dispositifs de réception pour amplifier et/ou filtrer les signaux reçus de la sonde 20, et des convertisseurs (convertisseurs analogique vers digital, et convertisseurs digital vers analogique) pour transformer les signaux en données représentatives du signal. Les données peuvent être enregistrées dans une mémoire de l'unité de calcul 30 et/ou directement traitées pour calculer des données intermédiaires (données de formation de voie ou autres). L'unité de calcul 30 peut implémenter tout procédé permettant ensuite de construire une image à partir des données des signaux reçus de la sonde 20, tel que la formation de voies.

L'image calculée peut être :

- une image du milieu (image B-mode) habituellement en niveau de gris pour visualiser des organes dans le milieu, et/ou
- une image montrant une vitesse ou un flux dans le milieu (image couleur) par exemple utile pour visualiser des vaisseaux sanguins et les flux associés dans le milieu, et/ou
- une image montrant une caractéristique mécanique du milieu (élasticité, selon le mode ShearWave® elastography) par exemple utile pour identifier des tumeurs à l'intérieur du milieu.

Par "connexion" ou "liaison" entre le dispositif de sondage 20, l'unité de calcul 30 et le dispositif d'affichage 50, on entend tout type de liaison filaire de type électrique ou optique, ou tout type de liaison sans fil utilisant tout protocole tel que le WiFi™, Bluetooth™ ou autres. Ces connexions ou liaisons sont à simple sens ou double sens. Le dispositif d'affichage 50 associé peut être de tout type, tel qu'un écran tactile ou non tactile, connecté ou pas.

Le dispositif d'affichage 50 est un écran permettant de visualiser l'image calculée par l'unité de calcul 30. Le dispositif d'affichage 50 peut aussi visualiser d'autres informations telles que les échelles de l'image, ou des informations de configuration pour le calcul ou traitement ou toute information de mesure ou d'aide. L'écran 50 peut être articulé sur un bras support 51 pour un meilleur positionnement pour l'utilisateur. L'écran 50 est usuellement un écran de grande taille (au moins 20 pouces) pour une meilleure visualisation pour l'utilisateur.

Le panneau de contrôle 40 est par exemple une portion d'un boitier système, ladite portion comprenant un boitier de panneau disposant d'une surface sensiblement plane 40a inclinée vers l'utilisateur pour manipulation d'une seule main. Comme représenté en **figure 3,** le panneau de contrôle 40 peut comprendre un écran de contrôle 49 pour visualiser diverses informations de configuration.

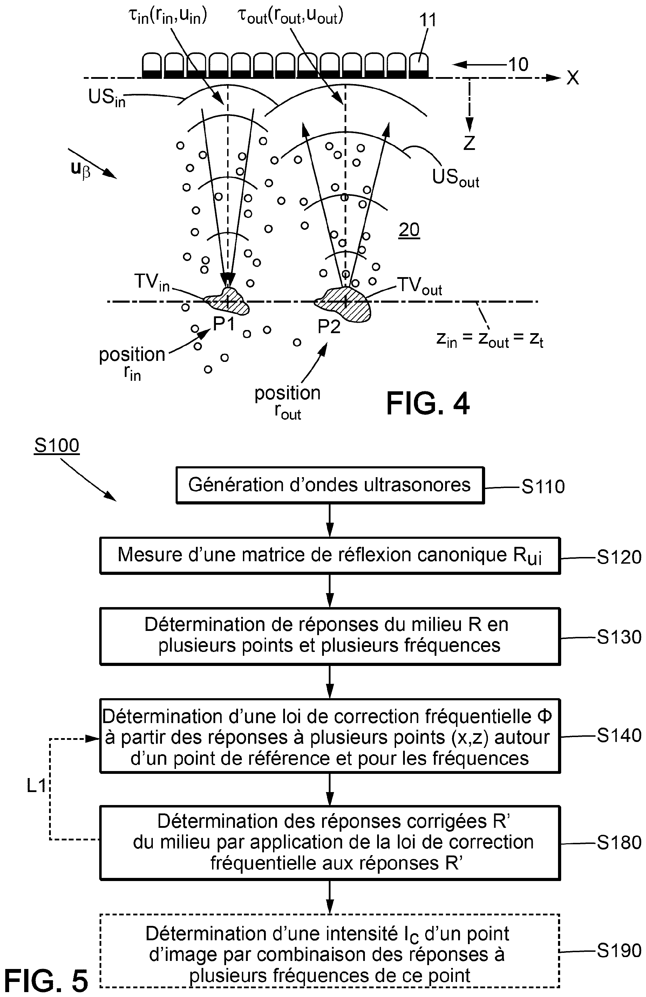

L'unité de calcul 30 est configurée pour la mise en oeuvre d'étapes de calculs et/ou traitement, notamment pour la mise en oeuvre d'étapes de procédés selon la présente divulgation. Par convention, comme représenté en **figure 4** montrant un réseau 10 de transducteurs 11 sur une surface d'un milieu M, on définit un repère spatial du milieu M, en prenant un premier axe X et un deuxième axe Z perpendiculaire à celui-ci. Par simplification, le premier axe X correspond à la direction transversale dans laquelle les transducteurs 11 sont alignés dans l'exemple d'un réseau linéaire, et le deuxième axe Z correspond à la profondeur du milieu M par rapport à ce réseau 10 de transducteurs 11. Cette définition peut être adaptée au contexte et ainsi par exemple étendue à un repère spatial à trois axes dans le cas d'un réseau 10 bidimensionnel, ou à un repère polaire dans le cas d'un réseau 10 courbé, ou à tout autre repère adapté et/ou dépendant de la structure et forme du réseau 10 de transducteurs ultrasonores. Ainsi, dans la suite de la présente divulgation, nous utiliserons un repère cartésien XZ, correspondant à une sonde 20 linéaire, pour plus de simplicité dans les explications, mais un spécialiste du domaine généraliserait et appliquerait facilement les résultats à tout type de repère.

Dans la suite de la divulgation, il est fait référence à un réseau 10 de transducteurs 11 pour l'émission et la réception, étant bien entendu que, dans un cas plus général, plusieurs réseaux de transducteurs pourront être utilisés simultanément. Les transducteurs 11 pourront être à la fois émetteur puis récepteur, ou bien seulement émetteur pour certains et seulement récepteur pour d'autres. De même, un réseau 10 peut être constitué d'un (1) à N traducteurs 11, de type identique ou de natures différentes.

Le réseau 10 de transducteurs 11 sert par exemple à la fois comme émetteur et comme récepteur, ou est constitué de plusieurs sous-réseaux de transducteurs, certains étant dédiés à l'émission, d'autres à la réception des ondes ultrasonores. Par réseau de transducteurs, on entend au moins un transducteur, une suite alignée ou non de transducteurs, ou une répartition bidimensionnelle de transducteurs (par exemple une matrice de transducteurs), ou toute répartition spatiale de transducteurs.

Lorsque dans la présente divulgation, il est fait référence à des étapes de calcul ou traitement pour la mise en oeuvre notamment d'étapes de procédés, il est entendu que chaque étape de calcul ou traitement peut être mis en oeuvre par logiciel, hardware, firmware, microcode ou toute combinaison appropriée de ces technologies ou technologies avoisinantes. Lorsqu'un logiciel est utilisé, chaque étape de calcul ou traitement peut être mise en oeuvre par des instructions de programme d'ordinateur ou du code qui peut être par exemple interprété, ou exécuté. Ces instructions peuvent être stockées ou transmises vers un support de stockage lisible par un ordinateur (ou unité de calcul) et/ou être exécutées par un ordinateur (ou unité de calcul) afin de mettre en oeuvre ces étapes de calcul ou traitement.

**Analyse d'un point du milieu par matrice de réflexion focalisée**

La présente divulgation décrit des procédés et systèmes de caractérisation ultrasonore d'un milieu. Dans les cas pratiques, le milieu est supposé hétérogène. Ces procédés et systèmes sont basés sur des définitions représentées en **figure 4** :

On définit dans le milieu :

- un premier point P1 de position spatiale $\mathbf{r_{in}}$ dans le repère spatial du milieu, et
- un deuxième point P2 de position spatiale $\mathbf{r_{out}}$ dans le repère spatial du milieu.

Ces positions spatiales $\mathbf{r_{in}}$ et $\mathbf{r_{out}}$ sont notées en gras, pour signifier que ces éléments sont des vecteurs de position, vecteurs pris dans le repère spatial du milieu (X, Z). D'autres représentations et définitions des positions des points sont possibles et accessibles à tout technicien des ultrasons.

Dans la présente divulgation, le premier point P1 a une position latérale notée, $x_{in}$. Le deuxième point P2 a une position latérale notée $x_{out}$. Les deux point P1, P2 ont la même profondeur attendue $z_{in} = z_{out}$, notée également z, qui est contrôlée par le temps d'échos $t$ considéré. Ainsi, les positions spatiales des points P1 et P2 sont respectivement $\mathbf{r_{in}} = (x_{in}, z)$ et Tout $= (x_{out}, z)$.

Ces deux points P1 et P2 sont choisis plutôt à faible distance l'un de l'autre, c'est à dire à quelques millimètres l'un de l'autre, et par exemple à vingt (20) millimètres ou moins.

Le procédé de caractérisation ultrasonore mis en oeuvre par l'unité de calcul 30 du système 40 comprend les étapes de :

- génération d'une série d'ondes ultrasonores incidentes $US_{in}$ dans une zone dudit milieu, au moyen d'un réseau 10 de transducteurs 11, ladite série d'ondes ultrasonores incidentes étant une base d'émission $\mathbf{i}$ ; et
- mesure ou construction d'une matrice de réflexion canonique $\mathbf{R_{ui}}(t)$ définie entre la base d'émission $\mathbf{i}$ en entrée et une base de réception $\mathbf{u}$ en sortie, les coefficients de cette matrice de réflexion canonique correspondant aux signaux reçus par les transducteurs et induits par les ondes ultrasonores réfléchies par des diffuseurs dans le milieu ;
- détermination d'une matrice de réflexion focalisée $\mathbf{R_{xx}}(z)$ comprenant des réponses du milieu calculées par focalisation à partir de la matrice de réflexion canonique $\mathbf{R_{ui}}(t)$ entre un transducteur virtuel d'entrée $TV_{in}$ de position spatiale $\mathbf{r_{in}}$ et un transducteur virtuel de sortie $TV_{out}$ de position spatiale $\mathbf{r_{out}}$.

La matrice de réflexion canonique $\mathbf{R_{ui}}(t)$ obtenue peut être une matrice « réelle », c'est-à-dire composée de coefficients réels dans le domaine temporel, les signaux électriques enregistrés par chacun des transducteurs étant des nombres réels. En variante, cette matrice peut être une matrice « complexe », c'est-à-dire composée de valeurs complexes, par exemple dans le cas d'une démodulation pour une formation de voies en phase et en quadrature (connu en langue anglaise sous la dénomination « beamforming IQ »).

La matrice de réflexion focalisée $\mathbf{R_{xx}}$ peut s'exprimer de différentes manières. Dans les expressions de la présente divulgation, le premier point P1 de position spatiale $(x_{in}, z)$ est pris comme référence. Ces expressions peuvent également être établies par rapport au deuxième point P2 de position spatiale $(x_{out}, z)$ ou par rapport à un point milieu entre P1 et P2, de position spatiale $((x_{in}+x_{out})/2, z)$ ou par rapport à tout point désigné comme point de référence. Le technicien du domaine pourra effectuer les changements de variables nécessaires dans les expressions présentées.

Les réponses du milieu sont calculées par focalisation à partir de la matrice de réflexion canonique $\mathbf{R_{ui}}(t)$.

Les réponses de la matrice de réflexion focalisée $\mathbf{R_{xx}}(z)$ correspondent à un champ de pression acoustique calculé entre tous les points du milieu de positions latérales $x_{in}$, et $x_{out}$, situés à la profondeur attendue $z$ et à un temps d'écho $t$, et pour une vitesse du son supposée $c_0$. Autrement dit, cette matrice de réflexion focalisée $\mathbf{R_{xx}}(z)$ est définie par :
$\mathbf{R_{xx}}(z) = [R(x_{in}, x_{out}, z)]$

Les paramètres que sont la profondeur $z$ dans le milieu, et la vitesse du son $c_0$ influent sur les lois de retard utilisées dans le processus de focalisation.

La base d'émission $\mathbf{i}$ en entrée est par exemple une base d'ondes générées chacune par un seul des transducteurs 11 du réseau 10 ou une base d'ondes planes d'inclinaison angulaire $\theta$ par rapport à l'axe X ou une base de sources virtuelles, comme décrit précédemment dans la divulgation des **figures 2(a) à 2(f).**

La base de réception $\mathbf{u}$ est par exemple la base des transducteurs 11. Dans une alternative, une autre base de réception peut être utilisée en réception, par exemple la base des ondes planes (ou base de Fourier spatiale), ou toute autre base permettant une mesure du champ ultrasonore dans un plan intermédiaire entre la sonde ultrasonore et le plan focal considéré par un processus adéquat de formation de voies en réception.

Ainsi, l'étape de génération des ondes ultrasonores s'entend entre la base d'émission $\mathbf{i}$ et la base de réception $\mathbf{u}$. Cette étape de génération ultrasonore est donc définie pour tout type d'ondes ultrasonores de type focalisées ou non focalisées, telles que des ondes planes.

Dans l'étape de mesure, la matrice de réflexion canonique $\mathbf{R_{ui}}(t)$ est définie entre la base d'émission $\mathbf{i}$ en entrée et une base de réception $\mathbf{u}$ en sortie. Cette matrice contient l'ensemble des réponses temporelles du milieu, mesurées au temps $t$ par chaque transducteur 11 de coordonnée spatiale $\mathbf{u_{out}}$ et pour chaque émission $\mathbf{i_{in}}$. On comprend que les éléments dénommés avec l'indice "in" font référence à l'émission (i.e. l'entrée) et les éléments dénommés avec l'indice "out" font référence à la réception (i.e. la sortie). Cette matrice canonique peut également être enregistrée et/ou stockée, par exemple en mémoire de l'unité de calcul, ou sur un tout autre support, amovible ou non, permettant un stockage permanent ou temporaire.

A l'étape de détermination de la matrice de réflexion focalisée $\mathbf{R_{xx}}(z)$ peut être obtenue par :

- un processus de focalisation en entrée à partir de la matrice de réflexion canonique $\mathbf{R_{ui}}(t)$ qui utilise un temps de vol à l'aller des ondes entre la base d'émission **i** et le transducteur virtuel en entrée $TV_{in}$ et qui crée une tache focale dite d'entrée autour du premier point P1 de position spatiale $\mathbf{r_{in}}$, ladite tache focale d'entrée correspondant au transducteur virtuel d'entrée $TV_{in}$,
- un processus de focalisation en sortie à partir de la matrice de réflexion canonique $\mathbf{R_{ui}}(t)$ qui utilise un temps de vol de retour des ondes entre le transducteur virtuel de sortie $TV_{out}$) et les transducteurs de la base de réception **u** et qui crée une tache focale dite de sortie autour du deuxième point P2 de position spatiale $\mathbf{r_{out}}$, ladite tache focale de sortie correspondant au transducteur virtuel de sortie $TV_{out}$.

Ces processus de focalisation en entrée et en sortie forment un processus de focalisation en entrée-sortie, dénommé dans la suite de cette divulgation, processus de focalisation ou plus simplement focalisation.

Autrement dit, dans ce procédé de caractérisation ultrasonore, le transducteur virtuel d'entrée $TV_{in}$ correspond à une "source virtuelle" ultrasonore située à la position spatiale $\mathbf{r_{in}}$ dans le milieu et le transducteur virtuel de sortie $TV_{out}$ correspond à un "capteur virtuel" ultrasonore situé à la position spatiale $\mathbf{r_{out}}$. Cette source et ce capteur virtuels sont spatialement séparés de la différence de leurs positions spatiales $\Delta\mathbf{r} = \mathbf{r_{out}} - \mathbf{r_{in}}$. Dans le cas présent, ils sont séparés uniquement selon un axe latéral, de $\Delta x = x_{out} - x_{in}$. Leur profondeur attendue est le paramètre $z$ utilisé dans la loi de focalisation pour un modèle de vitesse du son $c_0$. Leur profondeur réelle est dictée par la position axiale (en profondeur) du volume isochrone, c'est à dire par le temps d'écho $t$ et par la distribution de vitesse du son $c(\mathbf{r})$ dans le milieu. La dimension latérale des transducteurs virtuels est dictée par la tache focale produite par la focalisation à cette profondeur réelle.

En outre, la matrice de réflexion focalisée $\mathbf{R_{xx}}(z)$ peut être déterminée ou calculée :

- soit dans le domaine temporel, auquel cas elle peut être notée explicitement avec le paramètre de temps $t$, c'est-à-dire notée $\mathbf{R_{xx}}(z, t)$, et
  en pratique les données de la matrice de réflexion focalisée $\mathbf{R_{xx}}(z, t)$ sont calculées entre deux instants temporels prédéterminés ;
- soit dans le domaine fréquentiel, auquel cas elle peut être notée explicitement avec le paramètre de pulsation $\omega$ qui correspond à une fréquence $f$ par $\omega = 2\pi/f$, c'est-à-dire notée $\mathbf{R_{xx}}(z, \omega)$, et
  en pratique les données de la matrice de réflexion focalisée $\mathbf{R_{xx}}(z, \omega)$ sont calculées entre deux pulsations $\omega$, d'une bande passante fréquentielle, par exemple entre une pulsation inférieure $\omega-$ et une pulsation supérieure $\omega+$ , pour une pulsation centrale $\omega_c$.

Ainsi, la suite des calculs du procédé peut être effectué dans le domaine temporel ou dans le domaine fréquentiel.

Dans le premier cas de calcul dans le domaine temporel, la matrice de réflexion focalisée $\mathbf{R_{xx}}(z, t)$ du milieu entre le transducteur virtuel d'entrée $TV_{in}$ et le transducteur virtuel de sortie $TV_{out}$ est obtenue par focalisation par un calcul de formation de voies en entrées et en sortie. Les coefficients de cette matrice de réflexion focalisée $\mathbf{R_{xx}}(z, t)$ peuvent être déterminés par :

$$R(x_{in}, x_{out}, z, t)$$

$$= \frac{1}{N_{in}N_{out}} \sum_{\mathbf{i}_{in}} \sum_{\mathbf{u}_{out}} A_{in}(\mathbf{i}_{in}, x_{in}z) A_{out}(\mathbf{u}_{out}, x_{out}, z) R\big(\mathbf{i}_{in}, \mathbf{u}_{out}, t$$

$$+ \tau_{in}(\mathbf{i}_{in}, x_{in}, z) + \tau_{out}(\mathbf{u}_{out}, x_{out}, z)\big)$$

$$(1)$$

dans laquelle :

$N_{in}$ est un premier coefficient de normalisation,
$N_{out}$ est un deuxième coefficient de normalisation,
$\mathbf{R_{ui}}(t)$ est la matrice de réflexion confocale, dont
$R(u_{out}, i_{in}, t)$ est l'élément de la matrice de réflexion confocale $\mathbf{R_{ui}}(t)$ enregistré par le transducteur de position spatiale $\mathbf{u_{out}}$ consécutif à l'émission d'indice $\mathbf{i_{in}}$ dans la base d'émission **(i)** et au temps $t$, auquel on a appliqué les temps de

retard $\tau_{in}$ et $\tau_{out}$ ;

$A_{in}(\mathbf{i}_{in}, x_{in}z)$ et $A_{out}(\mathbf{u}_{out}, x_{out}, z)$ sont des coefficients d'apodisation qui sont prédéfinis, par exemple pour garder une ouverture numérique constante aussi bien à l'émission qu'à la réception ;

Le premier coefficient de normalisation $N_{in}$ peut par exemple être défini par : $N_{in}(x_{in}, z) = \Sigma_{i_{in}} A_{in}(\mathbf{i}_{in}, x_{in}, z)$ De même, le deuxième coefficient de normalisation $N_{out}$ peut être défini par : $N_{out}(x_{out}, z) = \Sigma_{u_{out}} A_{out}(\mathbf{u}_{out}, x_{out}, z)$

$\tau_{in}(\mathbf{i}_{in}, x_{in}, z)$ est le temps de vol attendu pour chaque onde incidente $\mathbf{i}_{in}$ pour atteindre le premier point focal de position spatiale $(x_{in}, z)$ dans un milieu modèle de vitesse du son $c_0$

$\tau_{out}(\mathbf{u}_{out}, x_{out}, z)$ est le temps de vol attendu pour une onde réfléchie depuis le deuxième point focal de position spatiale $(x_{out}, z)$ au transducteur de position $\mathbf{u}_{out}$.

Ces temps de retard $\tau_{in}$ et $\tau_{out}$ sont habituellement calculés par un homme du métier à partir d'un modèle établi de vitesse du son. Une hypothèse relativement simplificatrice consiste à faire l'hypothèse d'un milieu homogène avec une vitesse du son constante $c_0$ ans le milieu. Dans ce cas, les temps de vols sont directement obtenus à partir des distances entre les transducteurs de la sonde et les transducteurs virtuels. Ainsi, ces calculs de temps de retard sont fonction du type d'onde, de la vitesse du son supposée, et de la géométrie du réseau de transducteurs.

Le nombre d'éléments de la base d'émission $N_{in}$ est par exemple supérieur ou égal à un (1), et avantageusement supérieur ou égal à deux (2). Le nombre d'éléments de la base de réception $N_{out}$ est par exemple supérieur ou égal à deux (2).

La précédente formule de formation de voies est donc une double somme des réponses temporelles enregistrées dans la matrice de réflexion canonique $\mathbf{R_{ui}}$, une première somme selon la base d'émission $\mathbf{i}$ traduisant une focalisation à l'émission et une seconde somme selon la base de réception $\mathbf{u}$ liée à une focalisation en réception, ce calcul étant effectué pour les coordonnées spatiales des deux points P1 et P2, c'est-à-dire les points respectifs de positions spatiales attendues $\mathbf{r_{in}} = (x_{in}, z)$ et $\mathbf{r_{out}} = (x_{out}, z)$. Le résultat de cette formule de formation de voies est donc un signal temporel pour ces deux coordonnées spatiales $(\mathbf{r_{in}}, \mathbf{r_{out}})$ ou pour ces deux positions latérales $x_{in}, x_{out}$.

Enfin, la matrice de réflexion focalisée $\mathbf{R_{xx}}(z, t)$ exprimée dans le domaine temporel, peut être transformée dans le domaine fréquentiel en une matrice de réflexion focalisée $\mathbf{R_{xx}}(z, \omega)$ par une transformée de Fourier, c'est-à-dire par :

$$\mathbf{R}_{xx}(z, \omega) = \int_{-\infty}^{+\infty} dt \, \mathbf{R}_{xx}(z, t) e^{-j\omega t}$$

$$(2)$$

Cette transformée de Fourier peut être implémentée par tout type de transformée de Fourier discrète, normalisée ou non.

Dans le deuxième cas de calcul dans le domaine fréquentiel, la matrice de réflexion canonique $\mathbf{R_{ui}}(t)$ exprimée dans le domaine temporel, puisqu'elle est constituée des signaux reçus par les transducteurs, peut être transformée dans le domaine fréquentiel en une matrice de réflexion canonique $\mathbf{R_{ui}}(\omega)$ par une transformée de Fourier, c'est-à-dire par :

$$\mathbf{R}_{ui}(\omega) = \int_{-\infty}^{+\infty} dt \, \mathbf{R}_{ui}(t) e^{-j\omega t}$$

$$(3)$$

Cette transformée de Fourier peut être implémentée par tout type de transformée de Fourier discrète, normalisée ou non.

Ainsi, la matrice de réflexion focalisée $\mathbf{R_{xx}}(z)$ ou $\mathbf{R_{xx}}(z, \omega)$ du milieu peut être obtenue par focalisation par le calcul matriciel ci-dessous, sensiblement équivalent à la focalisation par la formation de voie temporelle explicitée précédemment, c'est-à-dire par le produit matriciel suivant :

$$\mathbf{R}_{xx}(z, \omega) = \mathbf{G}_{ux}^{\dagger}(z, \omega)\mathbf{R}_{ui}(\omega)\mathbf{P}_{ix}^{*}(z, \omega)$$

$$(4)$$

dans lequel

la matrice $\mathbf{R}_{ui}(\omega)$ est la transformée de Fourier de la matrice de réflexion canonique $\mathbf{R}_{ui}(t)$,

la matrice $\mathbf{G}_{ux}(z, \omega)$ est la matrice de passage de réception adaptée pour le passage de la base de réception $\mathbf{(u)}$ à la

base focalisée **(x)** à la profondeur z et à la pulsation $\omega$,

la matrice $\mathbf{P}_{ix}(z, \omega)$ est la matrice de passage d'émission adaptée pour le passage de la base d'émission **(i) « i »** à la base focalisée **(x)** à la profondeur z et à la pulsation $\omega$,

Les symboles * et † désignent respectivement les opérations matricielles de conjugaison et de transposition-conjugaison.

**Procédé de correction des aberrations**

Le procédé S100 selon la présente divulgation a pour but la correction des aberrations dans la caractérisation ultrasonore du milieu M, ces aberrations étant par exemple dues à des variations de structures dans le milieu qui induisent des variations de vitesse du son et des variations de réflectivité.

Le procédé S100 selon la présente divulgation est illustrée en **figure 5,** et ce procédé mis en oeuvre par l'unité de calcul 30 du système 40, comprend les étapes de :

- génération S110 d'une série d'ondes ultrasonores incidentes $US_{in}$ dans une zone dudit milieu, au moyen d'un réseau 10 de transducteurs 11, ladite série d'ondes ultrasonores incidentes étant une base d'émission **i** ; et
- mesure ou construction S120 d'une matrice de réflexion canonique $\mathbf{R}_{ui}(t)$ définie entre la base d'émission i en entrée et une base de réception **u** en sortie, les coefficients de cette matrice de réflexion canonique correspondant aux signaux reçus par les transducteurs et induits par les ondes ultrasonores réfléchies par des diffuseurs dans le milieu.

Ces étapes S110 et S120 correspondent aux étapes respectives de génération et de mesure, précédemment décrites.

Le procédé S100 selon la présente divulgation comprend en outre une étape de :

- détermination S130 d'un ensemble de réponses $R$ du milieu.

Cette étape S130 est similaire à l'étape de détermination d'une matrice de réflexion focalisée $\mathbf{R}_{xx}(z,f)$, mais cette étape s'en distingue par le fait que les réponses ne sont pas nécessairement organisées en matrice. Elles peuvent correspondre au seul signal confocal, i.e. seulement à la diagonale de la matrice de réflexion focalisée. Par ailleurs, ces réponses sont calculées en plusieurs fréquences $f$ et en plusieurs points de positions spatiales $\mathbf{r} = (x, z)$ d'une région (région d'analyse) située autour d'un point de référence de position spatiale $r_p = (x_p, z_p)$. Les réponses autour du point du point de référence à plusieurs fréquences permettent d'analyser cette région autour de ce point de référence ce qui permet d'effectuer une correction locale des aberrations et/ou de la dispersion et/ou des réverbérations induites par le milieu en amont du plan focal.

Notamment, le procédé comprend ainsi une étape de :

- détermination S130 d'un ensemble de réponses $R$ du milieu qui sont obtenues par un processus de focalisation pour plusieurs fréquences $f$ des signaux reçus des ondes ultrasonores réfléchies et pour plusieurs points de positions spatiales $\mathbf{r} = (x, z)$ d'une région autour d'un point de référence de position spatiale $r_p = (x_p, z_p)$ à partir de la matrice de réflexion canonique $\mathbf{R}_{ui}(t)$ pour un modèle de vitesse du son $c_0$.

Le procédé S100 selon la présente divulgation comprend alors un traitement de correction comprenant des étapes de :

- détermination S140 d'une loi de correction fréquentielle $\Phi$ par moyenne ou corrélation des réponses du milieu aux différents points de position spatiale (x, z) autour du point de référence, la loi de correction fréquentielle étant adaptée au point de référence et étant déterminée aux fréquences$f$,
- détermination S180 des réponses corrigées $R'$ du milieu par application de la loi de correction fréquentielle $\Phi$ aux réponses $R$ du milieu autour du point de référence et pour la pluralité des fréquences $f$.

**Grâce à ces dispositions,** le procédé permet avantageusement de sonder localement le milieu et de corriger la matrice de réflexion focalisée par rapport aux aberrations, notamment en déterminant une loi de correction pour chaque point du milieu M de position spatiale $\mathbf{r}_p$, et pour chaque fréquence $f$ de l'onde ultrasonore.

En outre, le procédé peut comprendre une étape de :

- détermination S190 d'une intensité $I_c$ d'un point de l'image échographique correspondant à un point de position spatiale $\mathbf{r} = (x, z)$ par combinaison des réponses corrigées à plusieurs fréquences $f$ de ce point de référence.

Notamment, cette intensité est calculée par somme quadratique des réponses corrigées *R'* au point de position spatiale **r** = *(x, z)* et pour au moins une partie ou toutes les fréquences *f* précédemment calculées. On peut par exemple limiter le calcul dans une bande de fréquence prédéterminée pour la caractérisation ultrasonore du milieu.

## 1 - Premier mode de réalisation

**Un premier mode de réalisation** du procédé S100 de la présente divulgation effectue des calculs matriciels en effectuant des processus de focalisation entre des points de transducteurs virtuels d'entrée et des points de transducteurs virtuels de sortie pouvant être distincts ou non l'un de l'autre. Ainsi, un grand nombre de réponses du milieu sont calculées ce qui permet des analyses très poussées et donc un traitement de correction spécifique pour la caractérisation du milieu M.

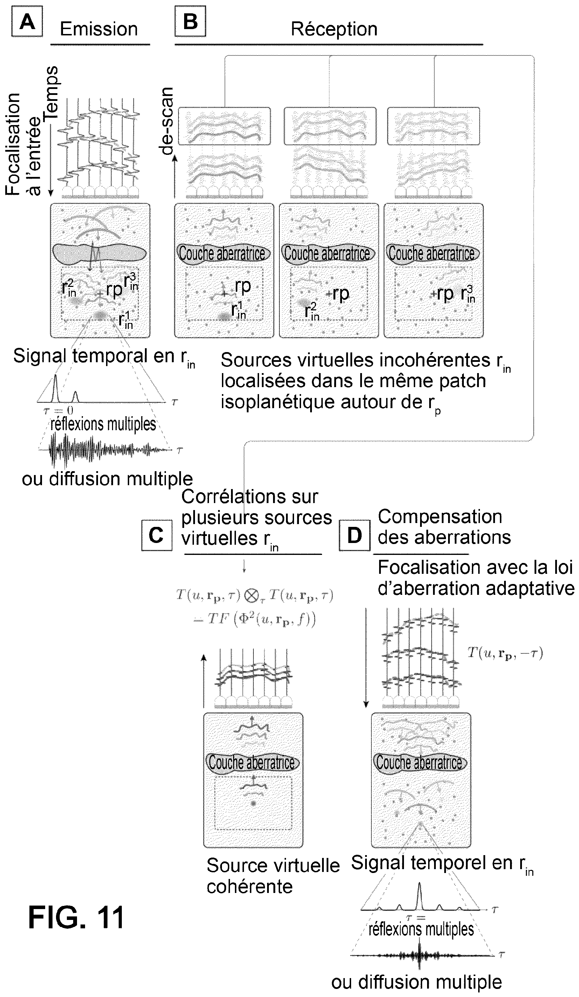

La **figure 11** illustre ce procédé selon le premier mode de réalisation de la présente divulgation. Le réseau de transducteurs, placé en vis-à-vis d'un milieu, permet d'insonifier et d'imager une région d'un milieu de réflectivité aléatoire type « speckle ».

Sur le premier schéma (A) de cette figure 11, une pluralité d'ondes sont émises successivement dans le milieu à l'aide d'émissions focalisées en direction de plusieurs points focaux de positions spatiales $r_{in}^1, r_{in}^2$ et $r_{in}^3$ par une technique dite de formation de voies ou focalisation. Les ondes traversent une couche aberratrice et induisent des taches focales distordues à la fois spatialement et temporellement autour des points focaux. La dispersion temporelle des ondes focalisées est induite ici par la dépendance fréquentielle de la vitesse du son au sein de la couche aberratrice et/ou des échos de réflexions multiples induits par cette dernière.

Sur les trois schémas suivants (B) de la figure, lors du trajet retour, les ondes réfléchies en chaque point focal traversent à nouveau la couche aberratrice, ce qui déforme de nouveau à la fois spatialement et temporellement les ondes ultrasonores, avec notamment une nouvelle multiplication d'échos induits par les réflexions multiples au sein de la couche aberratrice. Les signaux temporels reçus par les transducteurs sont donc très complexes et comprennent alors tous de nombreux échos liés aux réflexions multiples.

Comme représenté dans le cinquième schéma (C), en effectuant un traitement de moyenne ou de corrélation des échos induits par la pluralité des points focaux dans la région autour du point de référence de position spatiale $r_p$, on obtient une réponse temporelle telle qu'elle serait générée par un réflecteur cohérent virtuel. Ce calcul permet de déterminer une loi de correction spatio-fréquentielle (ici illustrée avec la base des transducteurs **u**).

Le sixième schéma (D) de cette figure explicite comment on peut utiliser cette réponse virtuelle déconvoluée et retournée temporellement comme loi de retard optimale à appliquer afin de focaliser correctement sur chaque point focal en compensant les problèmes d'aberrations et/ou de dispersion et/ou de réflexions multiples.

Le procédé de ce premier mode de réalisation est décrit plus en détail ci-après.

L'étape de détermination S130 d'un ensemble de réponses *R* du milieu comprend la détermination de réponses qui sont obtenues par le processus de focalisation entre un premier point de position spatiale $\mathbf{r_{in}}$ = *($x_{in}$, z)* correspondant à un transducteur virtuel d'entrée et un deuxième point de position spatiale $\mathbf{r_{out}}$ = *($x_{out}$, z)* correspondant à un transducteur virtuel de sortie, le premier point et le deuxième point étant situés dans la région autour du point de référence, et sont situés à la même profondeur *z,* les positions transverses $x_{in}$ et $x_{out}$ des premier et deuxième points formant une base focalisée **(x)** à chaque profondeur *z.*

Les réponses *R* sont alors par exemple enregistrées dans une matrice de réflexion focalisée $\mathbf{R_{xx}}(z, f)$ dont les coefficients s'écrivent ainsi selon $\mathbf{R_{xx}}(z, f) = [R(x_{in}, x_{out}, z, f)]$.

## Détermination de la loi de correction fréquentielle Φ (S140)

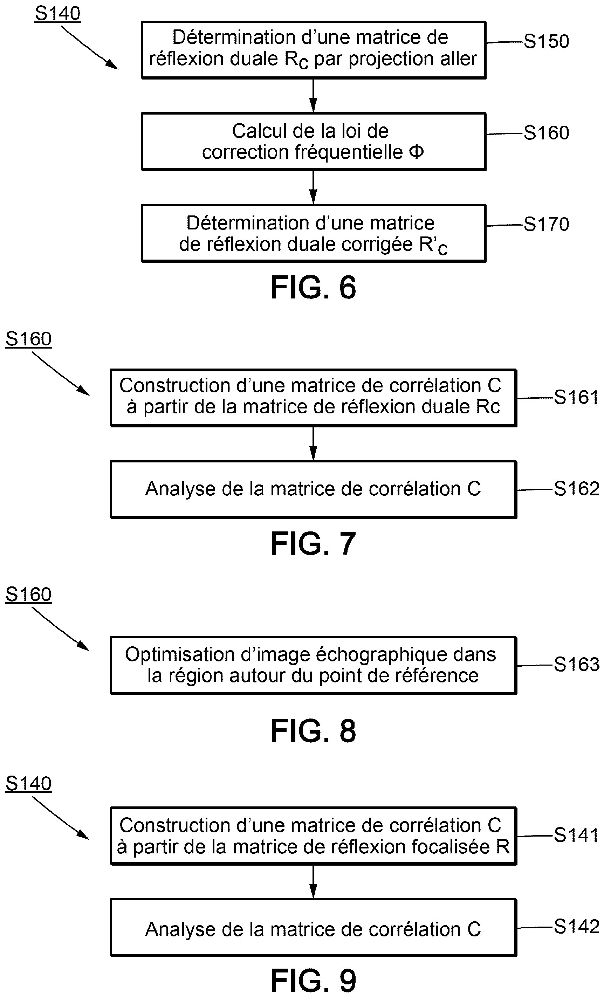

L'étape de détermination S140 d'une loi de correction fréquentielle Φ comme illustré en **figure 6,** comprend alors des sous-étapes effectuées à chaque profondeur *z* et chaque fréquence *f*, de :

- détermination S150 d'une matrice de réflexion duale $\mathbf{R_c}(z,f)$ par projection aller de la matrice de réflexion focalisée $\mathbf{R_{xx}}$ *(z,f)* vers une base de correction **(c),**
- calcul S160 de la loi de correction fréquentielle Φ à partir de la matrice de réflexion duale $\mathbf{R_c}(z,f)$, ladite loi de correction fréquentielle étant déterminée sur la base de correction **(c),** Φ = [$\phi(c, f, \mathbf{r_p})$], de sorte que ladite loi de correction fréquentielle Φ est une loi de correction spatio-fréquentielle,
- détermination S170 d'une matrice de réflexion duale corrigée $\mathbf{R'_c}$ autour du point de référence et dont les coefficients s'écrivent selon $\mathbf{R'_c}(z, f) = [R'_c(x, c, z, f)]$, déterminée en effectuant le produit terme à terme entre la matrice de réflexion duale $\mathbf{R}_c(z, f)$ et le conjugué en phase de la loi de correction fréquentielle $\Phi$, c'est-à-dire par :

$$\mathbf{R}_c' = \mathbf{R}_c \circ \Phi^*$$

où

le symbole * désigne une opération de conjugaison de phase
le symbole ∘ est le produit d'Hadamard, tel que :

$$R_c'(x, c, z, f) = R_c(x, c, z, f)\Phi^*(x, c, z, f).$$

L'étape de <u>détermination S180 des réponses corrigées</u> $R'$ du milieu autour du point de référence comprend alors la détermination d'une matrice de réflexion focalisée corrigée $\mathbf{R}_{xx}'(z, f)$ par projection retour de la matrice de réflexion duale corrigée $R'_c(z, f)$ vers la base focalisée **(x).**

**Grâce à ces dispositions,** le procédé permet avantageusement de sonder localement le milieu et de corriger la matrice de réflexion focalisée par rapport aux aberrations, notamment en déterminant une loi de correction pour chaque point du milieu M de position spatiale $\mathbf{r}_p$, et pour chaque fréquence $f$ de l'onde ultrasonore.

Cette correction est effectuée dans une base de correction **c** adaptée aux aberrations à corriger. La base de correction **c** est une base de correction en entrée ou une base de correction en sortie.

Des exemples de base de correction sont :

- une base des ondes planes ou base de Fourier spatiale,
- une base des transducteurs **u,**
- une base correspondant au lieu supposé des aberrateurs dans le milieu, c'est-à-dire par exemple un plan entre le plan des transducteurs (base des transducteurs **u**) et le plan de focalisation (base de focalisation **x**),
- une base correspondant à un plan déterminé par optimisation, par exemple par une matrice de corrélation dont la première valeur propre est maximale.

**Matrice de réflexion duale $\mathbf{R}_c(z, f)$ (S150)**

Selon un mode de réalisation du procédé de la présente divulgation, la projection aller S150 permet de déterminer une matrice de réflexion duale $\mathbf{R}_c(z, f)$. Cette projection aller S 150 peut être effectuée par :

un produit matriciel entre une matrice de passage **P** et la matrice de réflexion focalisée $\mathbf{R}_{xx}(z, f)$, c'est-à-dire :

$$\mathbf{R}_c(z, f) = \mathbf{P}(z, f) \times \mathbf{R}_{xx}(z, f)$$

où : $\mathbf{P}(z, f) = [P(c, x, z, f)]$ est la matrice de passage à chaque fréquence $f$ entre la base focalisée **(x)** à la profondeur $z$ et la base de correction **(c).**

La matrice de passage **P** dépend de la base de correction c utilisée.

Dans le cas d'une base de correction correspondant à une base des ondes planes (**c=k**), la matrice de passage **P** est l'opérateur de transformée de Fourier.

Dans le cas d'un réseau 10 de transducteurs 11 de type linéaire pour générer une image à deux dimensions, les coefficients de cette matrice de passage **P** peuvent s'écrire selon :

$$P(k_x, x, z, f) = P(k_x, x) = exp(-ik_x x)$$

où $k_x$, la composante transverse du vecteur d'onde **k** associé à chaque onde plane.

Dans le cas d'un réseau 10 de transducteurs 11 de type matriciel pour générer une image en trois dimensions, les coefficients de cette matrice de passage **P** peuvent s'écrire :

$$P(\mathbf{k}_{||}, \boldsymbol{\rho}, z, f) = P(\mathbf{k}_{||}, \boldsymbol{\rho}, z, f) = exp(-i\mathbf{k}_{||} \cdot \boldsymbol{\rho})$$

où

$k_\parallel$ est la composante transverse du vecteur d'onde **k** associé à chaque onde plane, et
$\rho = (x, y)$, le vecteur position transverse.

Dans le cas d'une base de correction correspondant à une base des transducteurs (**c=u**), les coefficients de la matrice de passage **P** correspondent à la dérivée normale de la fonction de Green reliant chaque point focal de position spatiale (*x, z*) et chaque transducteur 11 de position spatiale (*u, 0*).

Dans le cas d'un réseau 10 de transducteurs 11 de type linéaire pour générer une image à deux dimensions, les coefficients de la matrice de passage **P** peuvent s'écrire selon :

$$P(u, x, z, f) = \nabla_z G_{2D}(\mathbf{u}, \mathbf{r}, f)$$

où

$\nabla_z$ est le gradient projeté suivant la direction de profondeur *z*, et
$G_{2D}(\mathbf{u}, \mathbf{r})$ est la fonction de Green 2D qui relie chaque transducteur $\mathbf{u} = (u, 0)$ à chaque point **r** du milieu M , avec :

$$G_{2D}(\mathbf{u}, \mathbf{r}, f) = -\frac{i}{4}\mathcal{H}_0(k_0|\mathbf{u} - \mathbf{r}|)$$

où

$k_0 = 2\pi f/c_0$ est le nombre d'onde, $\mathcal{H}_0$
est la fonction de Hankel du 1^er ordre dont l'expression asymptotique est la suivante :

$$\mathcal{H}_0(2\pi f|\mathbf{u} - \mathbf{r}|/c_0) = e^{3i\pi/4}\sqrt{\frac{2}{\pi}}\frac{e^{-jk_0|\mathbf{u}-\mathbf{r}|}}{\sqrt{k_0|\mathbf{u}-\mathbf{r}|}}$$

Dans le cas d'un réseau 10 de transducteurs 11 de type matriciel pour générer une image en trois dimensions, les coefficients de cette matrice de passage **P** peuvent s'écrire :

$$P(u, x, z, f) = \nabla_z G_{3D}(\mathbf{u}, \mathbf{r}, f)$$

où $G_{3D}(\mathbf{u}, \mathbf{r})$ est la fonction de Green 2D qui relie chaque transducteur $\mathbf{u} = (u_x, u_y, 0)$ à chaque point $\mathbf{r} = (\rho, z)$ du milieu M, avec :

$$G_{3D}(\mathbf{u}, \mathbf{r}, f) = \frac{\exp\left(-ik_0\sqrt{|\mathbf{u}-\rho|^2+z^2}\right)}{4\pi\sqrt{|\mathbf{u}-\rho|^2+z^2}}$$

Les coefficients de la matrice de passage **P** s'écrivent donc dans ce cas de la manière suivante :

$$P(u, x, z, f) = -i\frac{fz}{c_0}\frac{\exp\left(-ik_0\sqrt{|\mathbf{u}-\rho|^2+z^2}\right)}{|\mathbf{u}-\rho|^2+z^2}$$

**Loi de correction spatio-fréquentielle (S160)**

Selon un premier mode de réalisation illustré en **figure 7** de l'étape de calcul de la loi de correction spatio-fréquentielle S160, cette étape de calcul S160 comprend :

la construction d'une matrice de corrélation S161 **C** à partir de la matrice de réflexion duale $\mathbf{R}_c(z,f)$, déterminée à l'étape de projection aller S140, et
l'analyse S162 de la matrice de corrélation **C** pour déterminer la loi de correction spatio-fréquentielle $\Phi$.

18

La loi de correction spatio-fréquentielle permet de corriger la matrice de réflexion duale $R_c(z,f)$ dans la base de correction **c**, pour obtenir une matrice de réflexion duale corrigée $R'_c(z,f)$. Cette correction dans la base de correction c est alors appliquée vers la base focalisée **x** par la projection retour S180 de la matrice de réflexion duale corrigée $R'_c(z,f)$ afin d'obtenir une matrice de réflexion focalisée corrigée $\mathbf{R}'_{xx}(z, f)$.

**Matrice de corrélation (S161)**

Selon une première variante de l'étape S161, la matrice de corrélation **C** est déterminée dans la base de correction **c** et dans le domaine fréquentiel, par le calcul suivant des éléments de la matrice de corrélation $\mathbf{C} = \mathbf{C_{cc}}$ :

$$C(\{c,f\}, \{c',f'\}) =$$
$$\sum_{x,z} R_c(x,c,z,f) R^*_{ref}(x,c,z,f) \ R^*_c(x,c',z,f') \ R_{ref}(x,c',z,f')$$

où

$R_c$ est la matrice de réflexion duale,
$R_{ref}$ est une matrice de réflexion modèle d'un milieu modèle dans lequel la vitesse du son est $c_0$ vitesse du son attendue du milieu et dans lequel un réflecteur plan est positionné à la profondeur $z$,
x, $z$ sont les coordonnées de points de la région autour du point $r_p$,
* est l'opérateur de conjugaison.

L'adjonction d'une matrice de référence $R_{ref}$ à la matrice de réflexion $R_c$ dans l'équation précédente permet de compenser la composante géométrique de la matrice de réflexion prédite par le modèle de vitesse du son $c_0$ et d'isoler les composantes distordues et réverbérées du front d'onde. Vue depuis le plan focal, cette opération revient à déplacer virtuellement chaque point de focalisation (x, z) à l'origine du repère. Pour un milieu de réflectivité aléatoire (speckle ultrasonore) et un nombre de points de focalisation (x, z) suffisants dans la région autour du point de référence de position spatiale $r_p$, la matrice de corrélation obtenue est équivalente à celle qui serait mesurée dans la base de correction pour un réflecteur virtuel cohérent dont l'étendue est définie par la tache focale induite par le processus de focalisation dans ladite région. Ce réflecteur virtuel constitue une étoile guide dont on va se servir plus loin pour déterminer une loi de focalisation spatio-temporelle optimale dans la région autour du point de référence $r_p$.

Selon une deuxième variante de l'étape S161, la matrice de corrélation **C** est déterminée dans la base de correction **c** et dans le domaine fréquentiel, par le calcul suivant des éléments de la matrice de corrélation $\mathbf{C} = \mathbf{C_{cc}}$ :

$$C(\{c,f\}, \{c',f'\}) = \sum_{x,z} D_c(x,c,z,f) \times D_c^{\ *}(x,c',z,f')$$

où

$D_c$ est une matrice de distorsion duale obtenue par

$$\mathbf{D}_c(z,f) = \mathbf{R}_c(z,f) \circ \mathbf{R}^*_{ref}(z,f),$$

pouvant s'exprimer en termes de calcul des coefficients de cette matrice par :

$$D_c(x,c,z,f) = R_c(x,c,z,f) R^*_{ref}(x,c,z,f).$$

où

$R_c$ est la matrice de réflexion duale,
$R_{ref}$ est une matrice de réflexion modèle d'un milieu modèle dans lequel la vitesse du son est $c_0$ vitesse du son attendue du milieu et dans lequel un réflecteur plan est positionné à la profondeur $z$,
x, $z$ sont les coordonnées de points de la région autour du point $r_p$,
* est l'opérateur de conjugaison.

Cette deuxième variante est équivalente à la première variante. Elle fait apparaitre un calcul intermédiaire de la matrice de distorsion duale $\mathbf{D}_c$. Elle est donc moins directe que la première variante, mais permet d'extraire directement une loi de focalisation par une simple décomposition en valeurs singulières de la matrice $\mathbf{D}_c$.

Par rapport aux travaux antérieurs qui ne considéraient qu'une matrice distorsion fenêtrée temporellement à la seule fréquence centrale, l'originalité de la matrice distorsion considérée ici est sa dépendance fréquentielle qui permet d'accéder à une loi de focalisation spatio-temporelle complexe allant au-delà d'une simple loi de retards temporels. Cela permet en effet de compenser avantageusement, en plus des aberrations classiques, les problèmes de réverbération et de dispersion fréquentielle.

Selon une troisième variante de l'étape S161, la matrice de corrélation $\mathbf{C}$ est déterminée dans une base des points d'image de position spatiale ($x$, $z$), par le calcul suivant des éléments de la matrice de corrélation $\mathbf{C} = \mathbf{C_{rr}}$ :

$$C(\{x, z\}, \{x', z'\}) =$$
$$\sum_{c,f} R_c(x, c, z, f) R_{ref}^*(x, c, z, f) \ R_c^*(x', c, z', f) \ R_{ref}(x', c, z', f)$$

où

$\mathbf{R}_c$ est la matrice de réflexion duale,
$\mathbf{R}_{ref}$ est une matrice de réflexion modèle d'un milieu modèle dans lequel la vitesse du son est $c_0$ vitesse du son attendue du milieu et dans lequel un réflecteur plan est positionné à la profondeur $z$,
$x$, $z$ sont les coordonnées des points de l'image dans la région autour du point $\mathbf{r}_p$,
* est l'opérateur de conjugaison.

L'adjonction d'une matrice de référence $\mathbf{R}_{ref}$ à la matrice de réflexion duale $\mathbf{R}_c$ dans l'équation précédente permet de compenser la composante géométrique de la matrice de réflexion prédite par le modèle de vitesse du son $c_0$ et d'isoler les composantes distordues et réverbérées du front d'onde. Vue depuis le plan focal, cette opération revient à déplacer virtuellement chaque point de focalisation ($x$, $z$) à l'origine du repère, formant ainsi un ensemble d'étoiles guides incohérentes dont la distribution d'amplitude est dictée par le support de la tache focale mais également modulée par la réflectivité aléatoire du milieu. Le calcul de la matrice de corrélation $\mathbf{C}$ dans la base focalisée permet de déterminer le déphasage entre chaque réalisation d'étoile guide dans le milieu et sera exploitée par la suite pour déterminer la manière dont ces différentes réalisations peuvent être rephasées entre elles et être combinées afin de pouvoir générer une étoile guide cohérente.

Selon une quatrième variante de l'étape S161, la matrice de corrélation C est déterminée dans une base des points d'image de position spatiale ($x$, $z$), par le calcul suivant des éléments de la matrice de correction $\mathbf{C} = \mathbf{C_{rr}}$ :

$$C(\{x, z\}, \{x', z'\}) = \sum_{c,f} D_c(x, c, z, f) \times D_c^*(x', c, z', f)$$

où $\mathbf{D}_c$ est une matrice de distorsion duale obtenue par

$$\mathbf{D}_c(z, f) = \mathbf{R}_c(z, f) \circ \mathbf{R}_{ref}^*(z, f),$$

pouvant s'exprimer en termes de calcul des coefficients de cette matrice par :

$$D_c(x, c, z, f) = R_c(x, c, z, f) R_{ref}^*(x, c, z, f).$$

où

$\mathbf{R}_c$ est la matrice de réflexion duale,
$\mathbf{R}_{ref}$ est une matrice de réflexion modèle d'un milieu modèle dans lequel la vitesse du son est $c_0$ vitesse du son attendue du milieu et dans lequel un réflecteur plan est positionné à la profondeur $z$,
$x$, $z$ sont les coordonnées de points de la région autour du point $\mathbf{r}_p$,
* est l'opérateur de conjugaison.

**Analyse (S162)**

Selon une première variante de l'étape S162, l'analyse de la matrice de corrélation $\mathbf{C}$ est effectuée par une décomposition en valeurs propres de la matrice de corrélation **C**, et la loi de correction spatio-fréquentielle Φ est le premier vecteur propre **U**$_1$ de la matrice de corrélation **C** dans la base de correction (c), c'est-à-dire **C** = **C**$_{cc}$.

La matrice de corrélation étant hermitienne (**C** = **C**$^\dagger$), ses valeurs propres sont réelles et positives.

La matrice de corrélation **C**$_{cc}$ peut ainsi s'écrire :

$$\mathbf{C_{cc}} = \sum_{\boldsymbol{p}} \sigma_p \mathbf{U}_p\, U_p^\dagger$$

ou en termes de coefficients matriciels :

$$C(\{c,f\},\{c',f'\}) = \sum_{\boldsymbol{p}} \sigma_p U_p(c,f) U_p^*(c',f')$$

avec

**U**$_p$ correspondant aux vecteurs propres de la matrice de corrélation **C**

$\sigma_p$ correspondant aux valeurs propres réelles et positives de la matrice de corrélation **C**$_{cc}$ rangées dans un ordre décroissant : $\sigma_1 > \sigma_2 > \cdots > \sigma_N$

On a alors la loi de correction spatio-fréquentielle Φ qui est égale au premier vecteur propre, i.e. Φ(**r**$_p$) = **U**$_1$ ; ou à sa version normalisée, Φ(**r**$_p$) = exp($j$arg{**U**$_1$}), i.e. une loi de correction spatio-fréquentielle dont les coefficients sont d'amplitude unité mais dont la phase est égale à celle de **U**$_1$ (le symbole arg{**X**} désigne la phase du vecteur **X** ; ou à une correction de type filtre inverse, Φ(**r**$_p$) = exp($j$arg{**U**$_1$})/|**U**$_1$|. La première option est à privilégier si on est en présence d'un mauvais rapport signal à bruit (filtre adapté). En général, la seconde option sera toutefois privilégiée afin que la correction n'agisse pas comme un filtre en amplitude mais permette la correction des seules distorsions de phase. Enfin la troisième option est pertinente quand le milieu aberrateur atténue de manière inhomogène certaines composantes et/ou fréquences du champ que l'on souhaite réhausser afin d'avoir un estimateur plus fidèle de la réflectivité *in fine.*

Selon une deuxième variante de l'étape S162, l'analyse de la matrice de corrélation **C** est effectuée par une décomposition en valeurs singulières de la matrice de distorsion duale **D**$_c$ définie ci-dessus en deuxième variante de l'étape S161. La décomposition en valeurs propres de la matrice de corrélation **C**$_{cc}$ réalisée dans la première variante de l'étape S162 est en effet équivalente à la décomposition en valeurs singulières (SVD) de la matrice de distorsion duale **D**$_c$ lorsque ses coefficients sont organisés selon la définition suivante :

$$\mathbf{D}_c = [D_c(\{c,f\},\{x,z\})]$$

La décomposition en valeurs singulières s'applique sur des matrices de forme rectangulaire, et appliquée à la matrice de distorsion duale **D**$_c$, elle s'écrit ainsi :

$$\mathbf{D}_c = \sum_{\boldsymbol{p}} \sqrt{\sigma_p}\, \mathbf{U}_p\, V_p^\dagger$$

ou en termes de coefficients matriciels :

$$D(\{c,f\},\{x,z\}) = \sum_{\boldsymbol{p}} \lambda_p U_p(c,f) V_p^*(x,z)$$

avec **U**$_p$ = [$U_p(c,f)$] correspondant aux vecteurs singuliers de la matrice de distorsion duale **D**$_c$ dans la base de correction, ou de manière équivalente, aux vecteurs propres de la matrice **C**$_{cc}$ tels que définis dans la première variante de l'étape S162

**V**$_p$ = [$V_p(x,z)$] correspondant aux vecteurs singuliers de la matrice de distorsion duale **D**$_c$ dans la base focalisée,

$\lambda_p$ correspondant aux valeurs singulières de la matrice de distorsion duale **D**$_c$ qui sont, par définition, égales à la racine carrée des valeurs propres $\sigma_p$ de la matrice de corrélation **C** telles que définies dans la première variante de l'étape S162 : $\sigma_p = \lambda_p^2$ .

Ainsi, la loi de correction spatio-fréquentielle Φ est égale au premier vecteur singulier de la matrice de distorsion

duale $\mathbf{D_c}$, i.e. $\Phi(\mathbf{r_p}) = \mathbf{U}_1$ ; ou à sa version normalisée, $\Phi(\mathbf{r_p}) = \exp(j\arg\{\mathbf{U}_1\})$, i.e. une loi de correction spatio-fréquentielle dont les coefficients sont d'amplitude unité mais dont la phase est égale à celle de $\mathbf{U}_1$ (le symbole arg{$\boldsymbol{X}$} désigne la phase du vecteur $\mathbf{X}$) ; ou à une correction de type filtre inverse, $\Phi(\mathbf{r_p}) = \exp(j\arg\{\mathbf{U}_1\})/|\mathbf{U}_1|$.

L'intérêt de la décomposition en valeurs singulières de la matrice de distorsion duale $\mathbf{D_c}$ par rapport à une décomposition en valeurs propres de la matrice de corrélation $\mathbf{C_{cc}}$ est la rapidité du calcul des algorithmes numériques de la décomposition en valeurs singulières.

Cette recherche de la loi de correction spatio-fréquentielle $\Phi$ est aussi équivalente à résoudre l'équation suivante :

$$a\Phi(\mathbf{r_p}) = \mathbf{C_{cc}} \times \Phi(\mathbf{r_p})$$

où $\times$ est le produit matriciel et a est une constante
de manière itérative par l'expression suivante, qui correspond à un calcul par retournement temporel itératif :

$$\Phi_{n+1}(\mathbf{r_p}) = \mathbf{C_{cc}} \times \Phi_n(\mathbf{r_p}),$$

avec $\Phi_0$ un front d'onde arbitraire, par exemple $\Phi_0 = [1 \cdots 1]^T$

Alors, la loi de correction spatio-fréquentielle $\Phi$ est obtenue par :

$$\Phi(\mathbf{r_p}) = \lim_{n \to \infty} \Phi_n(\mathbf{r_p}),$$

ou sa version normalisée :

$$\Phi(\mathbf{r_p}) = \exp\left(j\arg\left\{\lim_{n \to \infty} \Phi_n(\mathbf{r_p})\right\}\right),$$

ou sa version filtre inverse :

$$\Phi(\mathbf{r_p}) = \frac{\exp\left(j\arg\left\{\lim_{n \to \infty} \Phi_n(\mathbf{r_p})\right\}\right)}{\left|\lim_{n \to \infty} \Phi_n(\mathbf{r_p})\right|^{1/2}},$$

Pour $n \to \infty$, l'algorithme de retournement temporel itératif converge vers le même premier vecteur singulier $\mathbf{U}_1$ de la matrice $\mathbf{D_c}$. En pratique, il peut y avoir un intérêt à passer par un algorithme de retournement temporel itératif plutôt que par une SVD car il peut converger au bout de quelques itérations, d'où une plus grande rapidité de calcul.

Selon une troisième variante de l'étape S162, l'analyse de la matrice de corrélation $\mathbf{C_{cc}}$ est effectuée par la résolution de l'équation suivante :

$$\Phi(\mathbf{r_p}) = \exp(j\arg\{\mathbf{C_{cc}} \times \Phi(\mathbf{r_p})\})$$

de manière itérative par l'expression suivante, qui correspond à un calcul par retournement de phase itératif :

$$\Phi_{n+1}(\mathbf{r_p}) = \exp(j\arg\{\mathbf{C_{cc}} \times \Phi_n(\mathbf{r_p})\})$$

où $\times$ est le produit matriciel,
avec : $\Phi_0$ un front d'onde arbitraire, par exemple $\Phi_0 = [1 \cdots 1]^T$. Alors, la loi de correction spatio-fréquentielle $\Phi$ est obtenue par :

$$\Phi(\mathbf{r_p}) = \lim_{n \to \infty} \Phi_n(\mathbf{r_p}),$$

Ou sa version filtre inverse :

$$\Phi(\mathbf{r_p}) = \frac{\lim_{n\to\infty}\Phi_n(\mathbf{r_p})}{|\mathbf{C_{cc}}\times\lim_{n\to\infty}\Phi_n(\mathbf{r_p})|^{1/2}},$$

L'intérêt d'un algorithme de retournement de phase itératif par rapport aux alternatives précédentes est d'être un estimateur plus fiable de la phase de la loi de correction $\Phi(\mathbf{r_p})$ et donc d'accéder *in fine* à une meilleure compensation des distorsions de phase induites par l'aberrateur.

Selon une quatrième variante de l'étape S162, l'analyse de la matrice de corrélation $\mathbf{C_{rr}}$ est effectuée par la résolution de l'équation suivante :

$$\mathbf{W} = \exp(j\,\arg\{\mathbf{C_{rr}}\times\mathbf{W}\})$$

où $\times$ est le produit matriciel,
de manière itérative par l'expression suivante :

$$\mathbf{W}_{n+1}(\mathbf{r_p}) = \exp\big(j\,\arg\{\mathbf{C_{rr}}\times\mathbf{W}_n(\mathbf{r_p})\}\big)$$

où $\times$ est le produit matriciel,
avec $\mathbf{W}_0$ un front d'onde arbitraire, par exemple $\mathbf{W}_0 = [1 \cdots 1]^T$ ce qui permet d'obtenir le vecteur $\mathbf{W}$ suivant :

$$\mathbf{W} = \lim_{n\to\infty}\mathbf{W}_n$$

Ce vecteur $\mathbf{W} = [W(x, z)]$ défini dans la base focalisée contient la phase de chaque étoile guide incohérente synthétisée par focalisation dans la région autour du point de référence $\mathbf{r_p}$.

Le conjugué en phase de ce vecteur $\mathbf{W}$ peut alors être exploité pour rephaser chaque étoile virtuelle incohérente de sorte à pouvoir les recombiner de manière cohérente et obtenir ainsi un estimateur de la loi de correction spatio-fréquentielle $\Phi$ non biaisé par la réflectivité aléatoire du milieu. Mathématiquement, cette opération s'écrit de la manière suivante :

$$\phi(c, f, \mathbf{r_p}) = exp\left\{j\times arg\left\{\textstyle\sum_{x,z}D_c(x, c, z, f)W^*(x, c, z, f)\right\}\right\}$$

L'intérêt de cette approche par rapport à une SVD de la matrice distorsion $\mathbf{D_c}$ (deuxième variante de l'étape S162) ou de l'algorithme de retournement phase itératif (troisième variante de l'étape S162) est de converger vers une loi de correction la plus isoplanétique possible, i.e. efficiente pour chaque point de la région autour du point de référence $\mathbf{r_p}$.

**Loi de correction spatio-fréquentielle (S160)**

Selon un deuxième mode de réalisation illustré en **figure 8** de l'étape de calcul de la loi de correction spatio-fréquentielle S160, cette étape de calcul S160 est effectuée par un algorithme d'optimisation S163 maximisant l'intensité ou intensité confocale d'une image échographique dans la région $\Omega_p$ autour du point de référence de position spatiale $\mathbf{r_p}$.

Autrement dit, la loi de correction spatio-fréquentielle $\Phi$ est déterminée par la maximisation suivante :

$$\phi(c, f, \mathbf{r_p}) = \operatorname*{argmax}_{\phi(c, f, \mathbf{r_p})}\left\{\textstyle\sum_{(x,z)\in\Omega_p}\mathfrak{I}(x, z)\right\}$$

où $\mathfrak{I}(x, z)$ est l'intensité de l'image échographique dans la région $\Omega_p$ pour la loi de correction spatio-fréquentielle $\phi(c, f, \mathbf{r_p})$ appliquée en entrée et en sortie.

Cette image échographique est par exemple déterminée par une triple somme sur les valeurs de fréquences $f$, sur la base de correction en entrée $\mathbf{c}_{in}$ et sur la base de correction en sortie $\mathbf{c}_{out}$.

Avec, les définitions précédemment données, on peut par exemple avoir le calcul suivant :

$$\Im(x, z)$$

$$= \left| \sum_f \sum_{c_{in}} \sum_{c_{out}} \exp\left(-i\phi(c_{in}, f, \mathbf{r_p})\right) P^*(c_{in}, f, x, z) R(c_{in}, c_{out}, z, f) P^*(c_{out}, f, x, z) \exp\left(-i\phi(c_{out}, f, \mathbf{r_p})\right) \right|^2$$

Le précédent calcul inclut une matrice de réflexion duale $\mathbf{R_{cc}}(c_{in}, c_{out}, f)$ qui est obtenue par projection aller S150 dans une base de correction en entrée $\mathbf{c}_{in}$ et une base de correction en sortie $\mathbf{c}_{out}$, et dont les coefficients peuvent être exprimés par :

$$R(c_{in}, c_{out}, z, f) = \sum_{x_{in}} \sum_{x_{out}} P\left(c_{in}, f, x_{in}, z\right) R(x_{in}, x_{out}, z, f) P\left(c_{out}, f, x_{out}, z\right)$$

Cette méthode de détermination de la loi de correction spatio-fréquentielle $\Phi$ est itérative avec des calculs d'images échographiques. Même si ces images échographiques sont limitées à la région autour du point de référence de position spatiale $\mathbf{r}_p$, les itérations de l'algorithme d'optimisation peuvent être couteuses en temps de calcul.

Cependant, cette méthode présente l'avantage de déterminer de manière plus exacte la loi de correction spatio-fréquentielle $\Phi$, car elle prend en compte la réciprocité des corrections d'aberrations à appliquer sur le trajet aller et sur le trajet retour des ondes ultrasonores.

**Matrice de réflexion duale corrigée (S170)**

Selon le mode de réalisation du procédé de la présente divulgation, illustré en **figure 6,** la matrice de réflexion duale corrigée S170 $\mathbf{R}'_c(z, f) = [R'_c(x, c, f, z)]$ est déterminée en effectuant le produit terme à terme entre la matrice de réflexion duale $\mathbf{R}_c(z, f)$ et le conjugué en phase de la loi de correction spatio-fréquentielle $\Phi$, c'est-à-dire par :

$$\mathbf{R}'_c = \mathbf{R}_c \circ \mathbf{\Phi}^*$$

où l'exposant * représente l'opération de conjugaison de phase
le symbole ∘ est le produit d'Hadamard, c'est-à-dire le produit matriciel terme à terme de coefficients des matrices, tel que

$$R'_c(x, c, f, z) = R_c(x, c, f, z)\Phi^*(x, c, f, z).$$

**Matrice de réflexion corrigée (S180)**

Selon le mode de réalisation du procédé de la présente divulgation, une matrice de réflexion focalisée corrigée $\mathbf{R}'_{xx}(z, f)$ est alors déterminée par projection retour S180 de la matrice de réflexion duale corrigée $\mathbf{R}'_c(z, f)$ vers la base focalisée (**x**).

La projection retour est effectuée par un produit matriciel entre la matrice de passage **P** définie plus haut et la matrice de réflexion focalisée $\mathbf{R}'_c(z, f)$, c'est-à-dire :

$$\mathbf{R}'_{xx}(z, f) = \mathbf{P}^\dagger(z, f) \times \mathbf{R}'_c(z, f)$$

Où l'exposant † désigne l'opération matricielle de trans-conjugaison.

**Itérations du traitement de correction (L1)**

Selon le mode de réalisation du procédé de la présente divulgation illustré en **figure 5,** les étapes du traitement de correction, c'est-à-dire les étapes de :

- S140 de détermination de la loi de correction fréquentielle Φ, ladite étape S140 comprenant éventuellement les étapes S150 de détermination de la matrice de réflexion duale $\mathbf{R}_c(z, f)$, S160 de calcul de la loi de correction fréquentielle Φ, et S170 de détermination de la matrice de réflexion duale corrigée $\mathbf{R}'_c(z, f)$, et

- S180 de détermination de la matrice de réflexion focalisée corrigée $\mathbf{R}'_{xx}(z, f)$,

sont itérées plusieurs fois (deux fois ou plus de deux fois), comme cela est représenté par la boucle L1 de la **figure 5.**

A chaque itération, la projection aller de l'étape S150 utilise la matrice de réflexion focalisée corrigée $\mathbf{R}'_{xx}(z, f)$ obtenue durant la projection retour de l'étape S180 de l'itération précédente à la place de la matrice de réflexion focalisée $\mathbf{R}_{xx}(z,f)$.

Ainsi, à chaque itération, la loi de correction de spatio-fréquentielle est améliorée pour prendre en compte de mieux en mieux une ou plusieurs aberrations du milieu M.

Selon une première variante de ce processus itératif, à chaque itération de l'étape S150 de détermination de la matrice de réflexion duale $\mathbf{R}_c(z, f)$, on utilise une base de correction **c** différente, par exemple pour corriger des aberrations différentes localisées en des lieux différents du milieu.

Par exemple, le milieu M peut être discrétisé ou modélisé par une succession de couches selon la direction de la profondeur $z$, et les bases de correction **c** des itérations correspondent à des plans de ces couches successives. Autrement dit, on applique au cours des itérations des corrections correspondant à une pluralité d'aberrations du milieu M.

Par exemple, le milieu M peut être spatialement segmenté en domaines, de manière prédéterminée ou automatiquement sur la base d'une première image échographique du milieu M. Chaque itération du processus itératif, effectuera une correction sur une base de correction **c** correspondant à chaque domaine dudit milieu M.

Selon une deuxième variante de ce processus itératif, à chaque itération de l'étape S150 de détermination de la matrice de réflexion duale $\mathbf{R}_c(z, f)$, on utilise une projection aller soit vers une base de correction en entrée, soit vers une base de correction en sortie de la matrice de réflexion. Dans ce dernier cas, la projection de la matrice $\mathbf{R}_{xx}(z,f)$ vers la base de correction est effectuée de la manière suivante :

$$\mathbf{R}_c(z,f) = \mathbf{P}(z,f) \times \mathbf{R}^{\mathsf{T}}_{xx}(z,f)$$

où le symbole T désigne l'opération matricielle de transposition.

Dans la succession des itérations, on peut alterner entre l'utilisation d'une base de correction en entrée et une base de correction en sortie. Ainsi, la loi de correction de spatio-temporelle Φ est améliorée à chaque itération, et la correction des aberrations est améliorée.

Selon une troisième variante de ce processus itératif, à chaque itération de l'étape S150 de détermination de la matrice de réflexion duale $\mathbf{R}_c(z, f)$, on utilise une région autour du point de position spatiale $\mathbf{r}_p$ de taille de plus en plus petite au cours de ces itérations. Autrement dit, à chaque itération la taille de la région de calcul autour du point de référence $\mathbf{r}_p$ est réduite. Par taille de la région, on peut comprendre la largeur dans la direction $x$ ou la profondeur dans la direction $z$, ou les deux, ou toute autre convention de dimension de cette région, par exemple adaptée au mode de scanning du milieu M.

Ainsi, la loi de correction Φ est de mieux en mieux adaptée à une aberration localisée à proximité du point de référence de position spatiale $\mathbf{r}_p$.

**Image confocale (S190)**

Selon un mode de réalisation du procédé de caractérisation ultrasonore de la présente divulgation, le procédé comprend en outre une étape de :

- détermination S190 d'une intensité $I_c(x,z)$ d'un point d'image échographique de position spatiale $(x, z)$, à partir des coefficients diagonaux de la matrice de réflexion focalisée corrigée $\mathbf{R}'_{xx}(z,f)$ intégrés sur la bande passante des signaux ultrasonores, c'est-à-dire par la combinaison des réponses corrigées $R'$ du point à plusieurs fréquences $f$. On a ainsi par exemple le calcul suivant :

$$I_c(x,z) = \left| \sum_f R'_{xx}(x,x,z,f) \right|^2$$

L'intensité précédente déterminée en une pluralité de points permet de construire une image confocale du milieu M corrigée, qui correspond à une image échographique classique débarrassée des problèmes d'aberrations, de réverbérations et de dispersion fréquentielle de la vitesse du son dans le milieu étudié.

### 2 - Deuxième mode de réalisation

**Un deuxième mode de réalisation** du procédé S100 de la présente divulgation effectue des calculs plus directs, par exemple dans le but de déterminer principalement une caractérisation du milieu M de manière confocale. Ce mode de réalisation simplifié peut être utile pour déterminer plus simplement l'intensité $I_c$ d'un point d'image échographique, afin de déterminer plus rapidement une image échographique d'une zone d'intérêt du milieu M.

Dans ce deuxième mode de réalisation, les processus de focalisation sont déterminés ou calculés uniquement entre des points de transducteurs virtuels d'entrée et des points de transducteurs virtuels de sortie identiques. Cela revient à déterminer uniquement les composantes diagonales de la matrice de réflexion focalisée $\mathbf{R}_{xx}(z, f)$ du premier mode de réalisation, et à les enregistrer dans une matrice de réflexion confocale $\mathbf{R}(z, f)$, ce qui réduit drastiquement le nombre de réponses du milieu calculées.

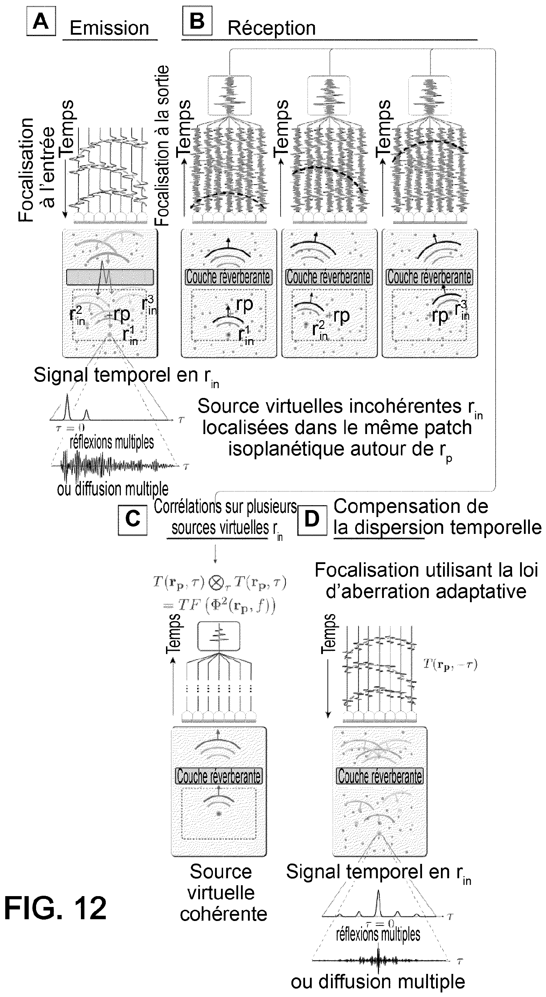

La **figure 12** illustre ce procédé selon le deuxième mode de réalisation de la présente divulgation. Le réseau de transducteurs, placé en vis-à-vis d'un milieu, permet d'insonifier et d'imager une région d'un milieu à réflectivité aléatoire type « speckle ».

Sur le premier schéma (A) de cette figure 11, une pluralité d'ondes sont émises successivement dans le milieu à l'aide d'émissions focalisées en direction de plusieurs points focaux de positions spatiales $r_{in}^1$, $r_{in}^2$ et $r_{in}^3$ par une technique dite de formation de voies ou focalisation. Les ondes traversent une couche réverbérante et arrivent vers les points focaux avec des échos de réflexions multiples induits par la couche réverbérante.

Sur les trois schémas suivants (B) de la figure, lors du trajet retour, les ondes réfléchies en chaque point focal traversent à nouveau la couche réverbérante, provoque une nouvelle multiplication d'échos induits par les réflexions multiples au sein de la couche réverbérante. Les signaux temporels reçus par les transducteurs sont très complexes et comprennent alors tous de nombreux échos liés aux réflexions multiples.

Comme représenté dans le cinquième schéma (C), en effectuant un traitement de moyenne ou de corrélation des échos induits par la pluralité des points focaux dans la région autour du point de référence de position spatiale $r_p$, on obtient une réponse temporelle telle qu'elle serait générée par un réflecteur cohérent virtuel. Ce calcul permet de déterminer une loi de correction fréquentielle à appliquer aux signaux pour compenser les réverbérations sur l'image échographique.

Le sixième schéma (D) de cette figure explicite comment on peut utiliser cette réponse virtuelle déconvoluée et retournée temporellement comme loi de retard optimale à appliquer afin de focaliser correctement sur chaque point focal en compensant les problèmes de dispersion temporelle et/ou de réflexions multiples.

Le procédé de ce deuxième mode de réalisation est décrit plus en détail ci-après.

Ce deuxième mode de réalisation du procédé S100 a les caractéristiques suivantes.

L'étape de détermination S130 d'un ensemble de réponses $R$ du milieu comprend la détermination de réponses qui sont obtenues par le processus de focalisation entre un premier point de position spatiale $r_{in} = (x_{in}, z)$ correspondant à un transducteur virtuel d'entrée et un deuxième point de position spatiale $r_{out} = (x_{out}, z)$ correspondant à un transducteur virtuel de sortie, le premier point et le deuxième point étant identiques ($r_{in} = r_{out}$), et l'ensemble des réponses $R$ étant enregistrés dans une matrice de réflexion confocale $\mathbf{R}$ dont les coefficients s'écrivent selon $\mathbf{R} = [R(x, z, f)]$.

Ainsi, en comparaison du premier mode de réalisation, la matrice de réflexion confocale $\mathbf{R}$ ne comprend plus qu'un seul paramètre de position latérale $x$, au lieu des deux paramètres indépendants de positions latérales $x_{in}$ et $x_{out}$.

L'étape de détermination (S140) de la loi de correction fréquentielle $\Phi$ est alors effectuée directement par corrélation des réponses du milieu aux différents points de positions spatiales $(x, z)$ autour du point de référence, et les coefficients de cette loi de correction fréquentielle s'écrivent $\Phi = [\phi(f, r_p)]$.

L'étape de détermination (S180) des réponses corrigées $R'$ autour du point de référence est effectuée alors directement par application de la loi de correction fréquentielle à chaque fréquence $f$, en effectuant le produit terme à terme entre la matrice de réflexion confocale $\mathbf{R}$ et le conjugué en phase de la loi de correction fréquentielle $\Phi$, c'est-à-dire par :

$$\mathbf{R}' = \mathbf{R} \circ \Phi^*$$

où

l'ensemble des réponses corrigées $R'$ sont enregistrées dans une matrice de réflexion confocale corrigée $\mathbf{R'}$ dont les coefficients s'écrivent selon $\mathbf{R'} = [R'(x, z, f)]$

le symbole ∘ est le produit d'Hadamard, tel que :

$$R'(x, z, f) = R(x, z, f)\phi^*(f, \mathbf{r_p}).$$

**Grâce à ces dispositions,** le procédé permet avantageusement de sonder localement le milieu et de corriger la matrice de réflexion confocale par rapport aux aberrations, notamment en déterminant directement une loi de correction pour des points de référence de position spatiale $\mathbf{r}_p$ du milieu M et pour plusieurs fréquences $f$ de l'onde ultrasonore.

La loi de correction fréquentielle $\Phi$ est calculée plus directement et plus simplement que dans le premier mode de réalisation, par corrélation des réponses reçues, c'est-à-dire sans utiliser de base de correction, ni de matrice de réflexion duale.

**Loi de correction fréquentielle (S140)**

Selon un premier mode de réalisation de l'étape de détermination d'une loi de correction fréquentielle S140, illustrée en **figure 9,** cette étape S140 comprend :

la construction d'une matrice de corrélation S141 **C** à partir de la matrice de réflexion confocale **R**($z$, $f$), et

une analyse S142 de la matrice de corrélation C pour déterminer la loi de correction fréquentielle $\Phi$.

Cette étape de détermination d'une loi de correction fréquentielle S140 est alors similaire à l'étape de calcul de la loi de correction fréquentielle S140 du premier mode de réalisation. Elle n'a pas besoin de matrice de réflexion duale et elle est directement mise en oeuvre sur la matrice de réflexion confocale. Les calculs sont ainsi simplifiés, mais les variantes possibles de ces étapes du procédé sont également similaires à celles du premier mode de réalisation, comme explicité ci-dessous.

Selon une première variante de l'étape S141, la matrice de corrélation C est déterminée dans le domaine fréquentiel par le calcul suivant :

$$C(f, f') = \sum_{x,z} R(x, z, f) \; R^*(x, z, f')$$

où

**R** est la matrice de réflexion confocale,
$x$, $z$ sont les coordonnées des points de la région autour du point de référence,
\* est l'opérateur de conjugaison.

Selon une deuxième variante de l'étape S 141, la matrice de corrélation C est déterminée dans une base des points d'image de position spatiale (x, z), par :

$$C(\{x, z\}, \{x', z'\}) = \sum_f R(x, z, f) \, R^*(x', z', f)$$

où

**R** est la matrice de réflexion confocale,
$x$, $z$ sont les coordonnées des points de l'image dans la région autour du point de référence,
\* est l'opérateur de conjugaison.

Selon une première variante de l'étape S142, l'analyse de la matrice de corrélation **C** est une décomposition en valeurs propres de la matrice de corrélation C et la loi de correction fréquentielle $\Phi$ est le premier vecteur propre **U₁** de la matrice de corrélation **C.**

Selon une deuxième variante de l'étape S142, l'analyse de la matrice de corrélation **C** est la résolution d'une équation faisant intervenir la matrice de corrélation **C** et la loi de correction fréquentielle $\Phi$, cette résolution d'équation correspondant à un retournement temporel itératif ou un retournement de phase itératif, de manière similaire à ce qui a été explicité dans le premier mode de réalisation.

Selon un deuxième mode de réalisation de l'étape de détermination d'une loi de correction fréquentielle S140, la détermination de la loi de correction fréquentielle est effectuée par un algorithme d'optimisation maximisant l'intensité confocale d'une image échographique dans la région autour du point de référence, de manière similaire à ce qui a été explicité dans le premier mode de réalisation.

**Itérations du traitement de correction (L1)**

Comme illustré en **figure 5,** les étapes du traitement de correction, c'est-à-dire les étapes de :

- S140 de détermination de la loi de correction fréquentielle Φ, et
- S180 de détermination de la matrice de réflexion confocale corrigée $\mathbf{R'}(z, f)$,

sont itérées plusieurs fois (deux fois ou plus de deux fois), comme cela est représenté par la boucle L1 de la **figure 5.**

A chaque itération, l'étape de détermination de la loi de correction fréquentielle de l'étape S140 utilise la matrice de réflexion confocale corrigée $\mathbf{R'}(z, f)$ obtenue durant l'étape de détermination des réponses corrigées de l'étape S180 de l'itération précédente à la place de la matrice de réflexion confocale $\mathbf{R}(z,f)$.

Ainsi, à chaque itération, la loi de correction fréquentielle est améliorée pour prendre en compte de mieux en mieux une ou plusieurs aberrations du milieu M.

A chaque itération de l'étape S140, on peut utiliser une région autour du point de référence de position spatiale $\mathbf{r}_p$ de taille de plus en plus petite au cours de ces itérations. Autrement dit, à chaque itération la taille de la région de calcul autour du point de référence $\mathbf{r}_p$ est réduite. Par taille de la région, on peut comprendre la largeur dans la direction $x$ ou la profondeur dans la direction $z$, ou les deux, ou toute autre convention de dimension de cette région, par exemple adaptée au mode de scanning du milieu M.

Ainsi, la loi de correction Φ est de mieux en mieux adaptée à une aberration localisée à proximité du point de référence de position spatiale $\mathbf{r}_p$.

**Image confocale (S190)**

Selon un mode de réalisation du procédé de caractérisation ultrasonore de la présente divulgation, le procédé comprend en outre une étape de :

- détermination S190 d'une intensité $I_c(x,z)$ d'un point d'image échographique de position spatiale *(x, z)* par combinaison des réponses corrigées $R'$ de ce point à plusieurs fréquences $f$. On a ainsi par exemple le calcul suivant :

$$I_c(x, z) = \left| \sum_f R'(x, z, f) \right|^2$$

L'intensité précédente déterminée en une pluralité de points permet de construire une image confocale du milieu M corrigée, qui correspond à une image échographique classique débarrassée des problèmes d'aberrations, de réverbérations et de dispersion fréquentielle de la vitesse du son dans le milieu étudié.

**Résultats sur un milieu expérimental calibré (dit « fantôme »)**

La **figure 13** illustre un milieu expérimental calibré dit « fantôme » générant un speckle ultrasonore avec deux inclusions cylindriques présentant une plus grande réflectivité que le milieu environnant ainsi qu'une pluralité de réflecteurs ponctuels (fils de nylon) arrangés linéairement suivant deux lignes, l'une horizontale et l'autre verticale. En dehors des réflecteurs ponctuels, la vitesse du son dans ce milieu est environ de 1540 m.s$^{-1}$. Sur ce fantôme est placé une couche de plexiglass correspondant à une couche aberratrice, ayant une vitesse du son d'approximativement 2750 m.s$^{-1}$. Sur cette couche est alors positionnée le réseau 10 de transducteurs 11 de la sonde ultrasonore. Le milieu est insonifié par une pluralité d'ondes planes de divers angles compris entre -40 et +40 degrés par rapport au plan des transducteurs. Les ondes ultrasonores dans un tel milieu traversent des milieux présentant des vitesses du son différentes et subissent des réflexions multiples (réverbérations) entre les interfaces de ces milieux avant d'atteindre les diffuseurs du milieu fantôme.

La **figure 14** illustre une image échographique obtenue dans une zone d'intérêt du milieu expérimental de la figure 13. Cette image échographique est obtenue avec une hypothèse de vitesse du son de 1540 m.s$^{-1}$, dans le volume du milieu insonifié. Cette image échographique illustre les problèmes d'aberration et réverbération induits par la couche de plexiglas sur l'image des réflecteurs ponctuels. Premièrement, l'image de chaque réflecteur ponctuel est étirée latéralement, ce qui illustre la dégradation de la résolution latérale de l'image à cause des différences entre le modèle de vitesse du son $c_0$ et la distribution réelle de la vitesse du son dans le milieu. Deuxièmement, l'image de chaque réflecteur ponctuel est répétée en direction de la profondeur z derrière chaque image balistique d'un réflecteur, ce qui illustre les réverbérations induites par les échos de réflexions multiples au sein de la couche de plexiglass. Par conséquent, l'image échographique est très perturbée avec des distorsions à la fois latérales et axiales de l'image échographique. Dans le cas de l'imagerie médicale, ces problèmes conduisent à un faible contraste, une faible résolution et à l'apparition d'artefacts dans l'image échographique, ce qui entrave gravement le diagnostic du praticien.

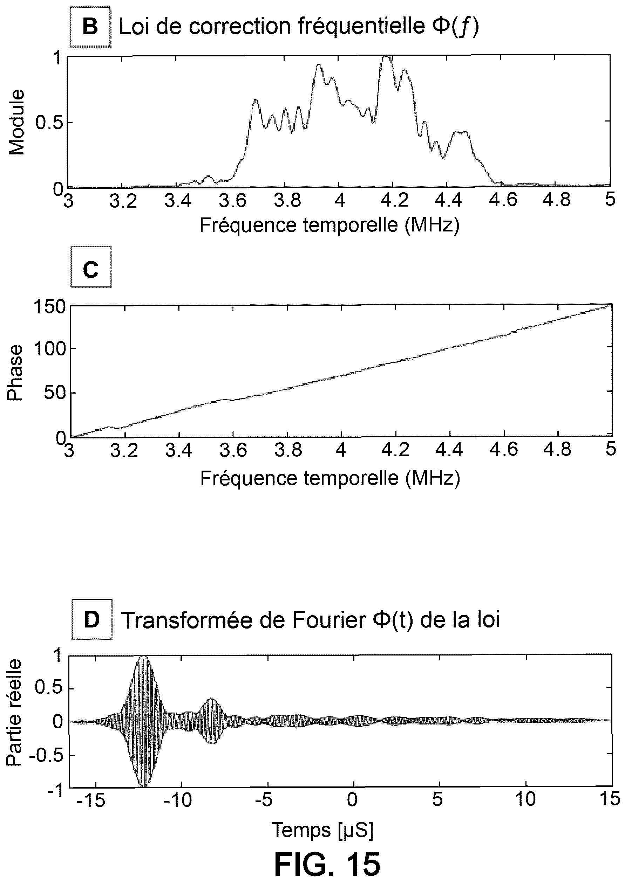

La **figure 15** illustre alors une loi de correction fréquentielle Φ obtenue par le procédé selon la présente divulgation dans la région du milieu B1 de la figure 14. Cette loi de correction fréquentielle est obtenue selon le deuxième mode de réalisation, par retournement de phase itératif de la matrice de corrélation fréquentielle C=[$C(f, f')$]des signaux confocaux de chaque point du milieu. Cette figure montre premièrement le module et la phase en fonction de la fréquence pour cette loi de correction fréquentielle, puis la représentation temporelle de cette loi de correction fréquentielle obtenue par transformée de Fourier inverse. La phase de la loi de correction fréquentielle correspond aux déphasages à appliquer à chaque composante fréquentielle des signaux ultrasonores. De manière équivalente, la loi de correction temporelle correspond à la loi des retards temporels qui doit être convoluée aux signaux ultrasonores reçus pour compenser (partiellement) les problèmes de réverbérations et obtenir une image échographique plus satisfaisante.

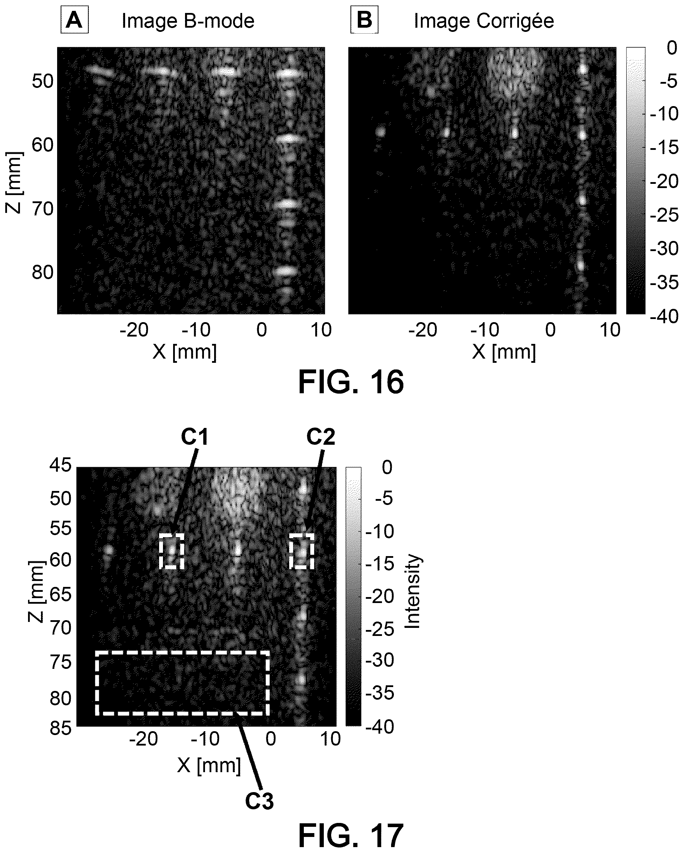

La représentation temporelle de la loi de correction fréquentielle comprend un premier écho de grande amplitude dont le temps d'écho inférieur au temps balistique correspond à l'erreur du modèle de propagation sur la vitesse du son dans le milieu, et plusieurs échos ultérieurs correspondant aux réflexions multiples dans la couche de plexiglass. Ainsi, cette loi de correction fréquentielle permet de corriger en moyenne les aberrations dans la région B1 du milieu expérimental qui correspond à une région de « speckle » du milieu. Cette loi est alors utilisée dans la zone d'intérêt de caractérisation du milieu, et notamment pour améliorer une image échographique corrigée de cette zone d'intérêt, comme cela est visible dans la figure 16(b) explicitée ci-dessous.

La **figure 16** illustre l'amélioration obtenue par le procédé selon la présente divulgation.

L'image de gauche (a) sur cette figure est l'image échographique obtenue avec le procédé de focalisation généralement utilisé dans l'art antérieur du milieu expérimental avec une hypothèse de vitesse du son $c_0$ de 1540 m.s$^{-1}$, et sans correction d'aberration. Cette image a une faible résolution et elle est de piètre qualité.

L'image de droite (b) sur cette figure est une image obtenue par le procédé décrit permettant de corriger des aberrations de vitesse du son et de réflexions multiples. On constate une grande amélioration sur la résolution spatiale de l'image des réflecteurs ponctuels dans le milieu et la suppression (partielle) des échos de réflexions multiples. Le fond d'image montre en outre une différence d'amplitude plus importante entre les réflecteurs virtuels et le speckle environnant. Le procédé de compensation des réverbérations permet donc d'améliorer singulièrement la qualité, en particulier le contraste de l'image échographique.

La **figure 17** illustre comme en figure 16(b) l'image échographique obtenue en regard d'une zone d'intérêt du milieu expérimental de la figure 13 après application d'une compensation des aberrations et réverbérations suivant le premier mode de réalisation, i.e en appliquant une correction spatio-fréquentielle Φ = [$\Phi(k_x, f)$] déterminée dans la base des ondes planes. Cette loi est obtenue par retournement de phase itératif de la matrice de corrélation spatio-fréquentielle $\boldsymbol{C_{kk}}$ = $C(\{k_x, f\}, \{k'_x, f'\})$ obtenue en moyennant les corrélations sur tous les points du champ de vision. Comme pour la figure 16(b), cette correction globale ne permet qu'une compensation partielle des aberrations et des réverbérations. A la différence de la figure 16(b), trois régions C1, C2, et C3 sont identifiées dans cette image afin d'appliquer localement et itérativement le procédé de la présente divulgation.

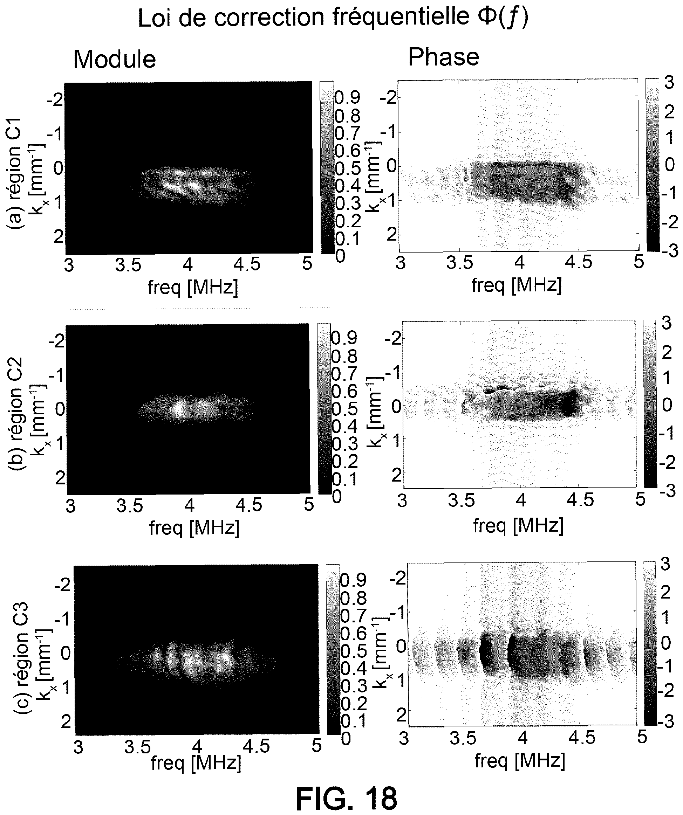

La **figure 18** illustre le résultat des déterminations de la loi de correction spatio-fréquentielle Φ dans la première région C1 de la figure 17, dans la région C2 de la figure 17, puis dans la région C3 de la figure 17. Ainsi, sur la première ligne (a) de cette figure 18, la loi de correction spatio-fréquentielle Φ dans la première région C1 est représentée par son spectre ($|\boldsymbol{C_{kk}} \times \Phi|$) en fonction de la composante transverse $k_x$ du vecteur d'onde et de la fréquence $f$ (image de gauche) et par sa phase (arg[Φ]) en fonction de $k_x$ et de la fréquence $f$ (image de droite). La deuxième ligne (b) de la figure 18, représente avec le même formalisme, la loi de correction spatio-fréquentielle Φ dans le deuxième région C2. Une autre loi de correction spatio-fréquentielle Φ est aussi déterminée pour la troisième région C3 dans le speckle ultrasonore.

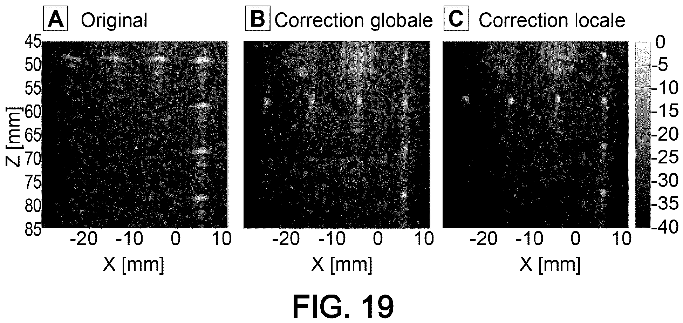

La **figure 19** illustre alors l'amélioration obtenue par le procédé selon la présente divulgation utilisant les lois de correction spatio-temporelles locales parmi lesquelles les corrections C1, C2 et C3.

L'image de gauche (a) sur cette figure est l'image échographique avec le procédé de focalisation généralement utilisé dans l'art antérieur du milieu expérimental avec une hypothèse de vitesse du son $c_0$ de 1540 m.s$^{-1}$, et sans correction d'aberration. Cette image a une faible résolution et elle est fortement dégradée par le phénomène de réverbérations.

L'image du centre (b) sur cette figure est une image obtenue par le procédé décrit permettant de corriger des aberrations de vitesse du son et de réflexions multiples de manière globale dans l'ensemble de la zone d'intérêt de l'image. Cette correction corrige en moyenne les aberrations pour toute cette zone d'intérêt, ce qui fournit déjà une amélioration. Cette image est analogue à celle montrée en figure 17.

L'image de droite (c) sur cette figure est une image obtenue par le procédé décrit en itérant les calculs de loi de correction spatio-fréquentielle de manière locale comme illustrée par la figure 18 pour les régions C1, C2 et C3 montrées sur la figure 17. Les corrections apportées sur les lois de correction spatio-fréquentielles permettent d'améliorer très notablement la résolution spatiale de l'image et permettent de supprimer en grande partie les échos induits par les réflexions multiples au sein de la couche de plexiglass. Enfin, l'amplitude relative entre les réflecteurs ponctuels et le speckle environnant est bien meilleur, ce qui illustre le gain en termes de contraste apporté par une compensation locale des aberrations et des réverbérations.

**Système de caractérisation ultrasonore**

**Le système 1 de caractérisation ultrasonore** du milieu M selon la présente divulgation est illustré en **figure 3.** Il comprend :

- un réseau 10 de transducteurs 11 adaptés pour générer une série d'ondes ultrasonores incidentes dans une zone du milieu, et pour mesurer en fonction du temps les ondes ultrasonores rétrodiffusées par ladite zone ; et
- une unité de calcul 30 reliée au réseau de transducteurs et adaptée pour mettre en oeuvre un procédé comprenant des étapes de :
- génération S110 d'une série d'ondes ultrasonores incidentes US$_{in}$ dans la zone du milieu, au moyen du réseau 10 de transducteurs 11, cette série d'ondes ultrasonores incidentes étant une base d'émission **i** ; et
- mesure S120 d'une matrice de réflexion canonique **R$_{ui}$**(t) définie entre la base d'émission **i** en entrée et une base de réception **u** en sortie, les coefficients de cette matrice de réflexion canonique correspondant aux signaux reçus par les transducteurs et induits par les ondes ultrasonores réfléchies dans le milieu ;
- détermination (S130) d'un ensemble de réponses $R$ du milieu qui sont obtenues par un processus de focalisation pour plusieurs fréquences $f$ et pour plusieurs points de position spatiale $r = (x, z)$ d'une région autour d'un point de référence de position spatiale $r_p = (x_p, z_p)$ à partir de la matrice de réflexion canonique **R$_{ui}$**(t) pour un modèle de vitesse du son $c_0$,
- détermination (S140) d'une loi de correction fréquentielle $\Phi$ à partir des réponses du milieu aux différents points de position spatiale ($x, z$), la loi de correction fréquentielle étant adaptée au point de référence et étant déterminée aux fréquences $f$,
- détermination (S180) des réponses corrigées $R'$ du milieu par application de la loi de correction fréquentielle $\Phi$ aux réponses $R$ du milieu autour du point de référence et pour la pluralité des fréquences $f$.

**Revendications**

**1.** **Procédé de caractérisation ultrasonore (S100)** d'un milieu, comprenant les étapes de :

- génération (5110) d'une série d'ondes ultrasonores incidentes (US$_{in}$) dans une zone d'intérêt dudit milieu, au moyen d'un réseau (10) de transducteurs (11), ladite série d'ondes ultrasonores incidentes étant une base d'émission (**i**) ; et
- mesure (S120) d'une matrice de réflexion canonique **R$_{ui}$**(t) définie entre la base d'émission (**i**) en entrée et une base de réception (**u**) en sortie, les coefficients de cette matrice de réflexion canonique correspondant aux signaux reçus par les transducteurs et induits par les ondes ultrasonores réfléchies dans le milieu ;

ledit procédé étant **caractérisé en ce qu'**il comprend en outre un traitement de correction comprenant les étapes de :

- détermination (S130) d'un ensemble de réponses $R$ du milieu qui sont obtenues par un processus de focalisation pour plusieurs fréquences $f$ des signaux reçus des ondes ultrasonores réfléchies et pour plusieurs points de position spatiale $r = (x, z)$ d'une région autour d'un point de référence de position spatiale $r_p = (x_p, z_p)$ à partir de la matrice de réflexion canonique **R$_{ui}$**(t) pour un modèle de vitesse du son $c_0$,
- détermination (S140) d'une loi de correction fréquentielle $\Phi$ par moyenne ou corrélation des réponses du milieu aux différents points de position spatiale ($x, z$) autour du point de référence, la loi de correction fréquentielle étant adaptée au point de référence et étant déterminée aux fréquences $f$,
- détermination (S180) des réponses corrigées $R'$ du milieu par application de la loi de correction fréquentielle $\Phi$ aux réponses $R$ du milieu autour du point de référence et pour la pluralité des fréquences $f$.

**2.** Procédé selon la revendication 1, comprenant en outre une étape de :

- détermination (S190) d'une intensité $I_c$ d'un point d'image échographique correspondant au point de référence de position spatiale **r**$_p$ par combinaison des réponses corrigées à plusieurs fréquences $f$ de ce point de référence.

**3.** Procédé selon la revendication 1 ou la revendication 2, dans lequel :

- la détermination (S130) de l'ensemble des réponses $R$ du milieu comprend la détermination de réponses qui sont obtenues par le processus de focalisation entre un premier point de position spatiale **r$_{in}$** = ($x_{in}, z$) correspondant à un transducteur virtuel d'entrée et un deuxième point de position spatiale **r$_{out}$** = ($x_{out}, z$) correspondant à un transducteur virtuel de sortie, le premier point et le deuxième point étant identiques (**r$_{in}$** = **r$_{out}$**),

et

l'ensemble des réponses $R$ étant enregistrés dans une matrice de réflexion confocale **R** dont les coefficients s'écrivent selon **R** = [$R(x, z, f)$],

- la détermination (S 140) de la loi de correction fréquentielle Φ est effectuée par corrélation des réponses du milieu aux différents points de positions spatiales ($x$, $z$) autour du point de référence, et dont les coefficients s'écrivent Φ = [$\phi(f, \mathbf{r}_p)$]

- la détermination (S180) des réponses corrigées $R'$ autour du point de référence, par application de la loi de correction fréquentielle à chaque fréquence $f$, en effectuant le produit terme à terme entre la matrice de réflexion confocale **R** et le conjugué en phase de la loi de correction fréquentielle Φ, c'est-à-dire par :

$$\mathbf{R}' = \mathbf{R} \circ \mathbf{\Phi}^*$$

où

l'ensemble des réponses corrigées $R'$ sont enregistrées dans une matrice de réflexion confocale corrigée **R'** dont les coefficients s'écrivent selon **R'** = [$R'(x, z, f)$]

le symbole ∘ est le produit d'Hadamard, tel que :

$$R'(x, z, f) = R(x, z, f)\phi^*(f, \mathbf{r}_p).$$

**4.** Procédé selon la revendication 3, comprenant en outre une étape de :

- détermination (S190) d'une intensité $I_c$ d'un point d'image échographique de position spatiale ($x$, $z$) par combinaison des réponses corrigées $R'$ de ce point à plusieurs fréquences $f$, c'est-à-dire par :

$$I_c(x, z) = \left| \sum_f R'(x, z, f) \right|^2$$

**5.** Procédé selon la revendication 3 ou la revendication 4, dans lequel :
la détermination d'une loi de correction fréquentielle (S140) comprend

la construction d'une matrice de corrélation (S141) **C** à partir de la matrice de réflexion confocale R($z$,f), et une analyse (S142) de la matrice de corrélation C pour déterminer la loi de correction fréquentielle Φ.

**6.** Procédé selon la revendication 5, dans lequel :
l'analyse (S142) de la matrice de corrélation **C** est une décomposition en valeurs propres de la matrice de corrélation C et la loi de correction fréquentielle Φ est le premier vecteur propre **U₁** de la matrice de corrélation **C.**

**7.** Procédé selon la revendication 5, dans lequel :
l'analyse (S 142) de la matrice de corrélation **C** est la résolution d'une équation faisant intervenir la matrice de corrélation **C** et la loi de correction fréquentielle Φ**,** cette résolution d'équation correspondant à un retournement temporel itératif ou un retournement de phase itératif.

**8.** Procédé selon l'une des revendications 3 à 7, dans lequel :
la détermination (S140) de la loi de correction fréquentielle est effectuée par un algorithme d'optimisation maximisant l'intensité confocale d'une image échographique dans la région autour du point de référence.

**9.** Procédé selon l'une des revendications 3 à 8, dans lequel :

les étapes (S140, S180) du traitement de correction sont itérées plusieurs fois, et
dans lequel à chaque itération, on utilise la matrice de réflexion confocale corrigée **R'**($z$,f) obtenue à l'itération précédente à la place de la matrice de réflexion focalisée **R**($z$,f).

**10.** Procédé selon la revendication 11, dans lequel :
à chaque itération, la taille de la région autour du point de référence de position spatiale $\mathbf{r}_p$ est réduite

**11.** Procédé selon la revendication 1 ou la revendication 2, dans lequel :

- la détermination (S130) de l'ensemble des réponses *R* du milieu comprend la détermination de réponses qui sont obtenues par le processus de focalisation entre un premier point de position spatiale $\mathbf{r_{in}}$= *(x$_{in}$, z)* correspondant à un transducteur virtuel d'entrée et un deuxième point de position spatiale $\mathbf{r_{out}}$ = *(x$_{out}$, z)* correspondant à un transducteur virtuel de sortie, le premier point et le deuxième point étant situés dans la région à la même profondeur *z*, les positions transverses *x$_{in}$* et *x$_{out}$* des premier et deuxième points formant une base focalisée (**x**) à chaque profondeur *z*, et

l'ensemble des réponses *R* étant enregistrés dans une matrice de réflexion focalisée $\mathbf{R_{xx}}$(z,f) dont les coefficients s'écrivent selon $\mathbf{R_{xx}}$(z, f) = [R(*x$_{in}$, x$_{out}$, z, f*)],

- la détermination (S140) de la loi de correction fréquentielle Φ comprend des sous-étapes effectuées à chaque profondeur *z* et chaque fréquence *f*, de :

- détermination (S150) d'une matrice de réflexion duale $\mathbf{R_c}$(z,f) par projection aller de la matrice de réflexion focalisée $\mathbf{R_{xx}}$(z,f) vers une base de correction (**c**),
- calcul (S160) de la loi de correction fréquentielle Φ à partir de la matrice de réflexion duale $\mathbf{R_c}$(z,f), ladite loi de correction fréquentielle étant déterminée sur la base de correction (**c**), Φ = [$\phi$(*c, f,* $\mathbf{r_p}$)], de sorte que ladite loi de correction fréquentielle Φ est une loi de correction spatio-fréquentielle,

- détermination (S170) d'une matrice de réflexion duale corrigée $\mathbf{R'_c}$ autour du point de référence et dont les coefficients s'écrivent selon $\mathbf{R'_c}(z,f) = [R'_c(x, c, f, z)]$, déterminée en effectuant le produit terme à terme entre la matrice de réflexion duale $\mathbf{R_c}$(z, f) et le conjugué en phase de la loi de correction fréquentielle Φ, c'est-à-dire par :

$$\mathbf{R'_c} = \mathbf{R_c} \circ \mathbf{\Phi^*}$$

où

le symbole * désigne une opération de conjugaison de phase
le symbole ∘ est le produit d'Hadamard, tel que :

$$R'_c(x, c, f, z) = R_c(x, c, f, z)\Phi^*(x, c, f, z)$$

- la détermination (S180) des réponses corrigées *R'* du milieu autour du point de référence comprend la détermination d'une matrice de réflexion focalisée corrigée $\mathbf{R'_{xx}}(z, f)$ par projection retour de la matrice de réflexion duale corrigée $\mathbf{R'_c}$(z,f) vers la base focalisée (**x**).

**12.** Procédé selon la revendication 11, dans lequel :
Le calcul de la loi de correction fréquentielle (S160) est effectué par un algorithme d'optimisation maximisant l'intensité confocale d'une image échographique dans la région autour du point de référence.

**13.** Procédé selon l'une des revendications 11 à 12, dans lequel :

les étapes (S140, S180) du traitement de correction sont itérées plusieurs fois, et
dans lequel à chaque itération, la projection aller (S150) utilise la matrice de réflexion focalisée corrigée $\mathbf{R'_{xx}}$(z,f) obtenue durant la projection retour (S180) de l'itération précédente à la place de la matrice de réflexion focalisée $\mathbf{R_{xx}}$(z,f).

**14.** Procédé selon la revendication 13, dans lequel :
à chaque itération, la taille de la région autour du point de référence de position spatiale $\mathbf{r_p}$ est réduite

**15. Système (1) de caractérisation ultrasonore d'un milieu (M),** le système comprenant :

- un réseau (10) de transducteurs adaptés pour générer une série d'ondes ultrasonores incidentes dans une zone d'intérêt du milieu, et pour mesurer en fonction du temps les ondes ultrasonores rétrodiffusées par ladite zone d'intérêt ; et
- une unité de calcul (30) reliée au réseau de transducteurs et adaptée pour mettre en oeuvre le procédé selon l'une des revendications 1 à 14.

**Canonique**

Emission     Réception

**Focalisée**

Emission     Réception

Temps $t$

Sonde $u$

(a)     (b)     (c)     (d)

**Ondes planes**

Emission     Réception

**Sources virtuelles**

Emission     Réception

(e)     (f)     (g)     (h)

**FIG. 1**

**A**  Imagerie **confocale**

**B**  Imagerie **matricielle**

Sonde $u$

entrée  sortie

entrée  sortie

Profondeur $z$

$x_{in} = x_{out}$  Transverse $x$

$x_{in}$  $x_{out}$

## FIG. 2

FIG. 3

FIG. 4

S100

| Génération d'ondes ultrasonores | —S110 |

| Mesure d'une matrice de réflexion canonique $R_{ui}$ | —S120 |

| Détermination de réponses du milieu R en plusieurs points et plusieurs fréquences | —S130 |

| Détermination d'une loi de correction fréquentielle $\Phi$ à partir des réponses à plusieurs points (x,z) autour d'un point de référence et pour les fréquences | —S140 |

L1

| Détermination des réponses corrigées R' du milieu par application de la loi de correction fréquentielle aux réponses R' | —S180 |

| Détermination d'une intensité $I_C$ d'un point d'image par combinaison des réponses à plusieurs fréquences de ce point | —S190 |

FIG. 5

S140

| Détermination d'une matrice de réflexion duale $R_C$ par projection aller |
|---|

S150

↓

| Calcul de la loi de correction fréquentielle Φ |
|---|

S160

↓

| Détermination d'une matrice de réflexion duale corrigée R'$_C$ |
|---|

S170

## FIG. 6

S160

| Construction d'une matrice de corrélation C à partir de la matrice de réflexion duale Rc |
|---|

S161

↓

| Analyse de la matrice de corrélation C |
|---|

S162

## FIG. 7

S160

| Optimisation d'image échographique dans la région autour du point de référence |
|---|

S163

## FIG. 8

S140

| Construction d'une matrice de corrélation C à partir de la matrice de réflexion focalisée R |
|---|

S141

↓

| Analyse de la matrice de corrélation C |
|---|

S142

## FIG. 9

**FIG. 10**

FIG. 11

**FIG. 12**

$(u)$

$h$

Balistique

Réflexions multiples

FIG. 13

A  Image B-mode

B1

FIG. 14

**B** Loi de correction fréquentielle $\Phi(f)$

**C**

**D** Transformée de Fourier $\Phi(t)$ de la loi

FIG. 15

A  Image B-mode   B  Image Corrigée

FIG. 16

FIG. 17

FIG. 18

FIG. 19

Europäisches Patentamt
European Patent Office
Office européen des brevets

# RAPPORT DE RECHERCHE EUROPEENNE

**Numéro de la demande**

EP 24 21 5535

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | LAMBERT WILLIAM ET AL: "Ultrasound Matrix Imaging-Part II: The Distortion Matrix for Aberration Correction Over Multiple Isoplanatic Patches", IEEE TRANSACTIONS ON MEDICAL IMAGING, IEEE, USA, vol. 41, no. 12, 16 août 2022 (2022-08-16), pages 3921-3938, XP011929114, ISSN: 0278-0062, DOI: 10.1109/TMI.2022.3199483 [extrait le 2022-08-17] | 1,2,15 | INV. G01S7/52 G01S15/89 |
| A | * le document en entier * ----- | 3-14 | |
| A,D | WO 2020/016250 A1 (CENTRE NAT RECH SCIENT [FR] ET AL.) 23 janvier 2020 (2020-01-23) * le document en entier * ----- | 1-15 | |

**DOMAINES TECHNIQUES RECHERCHES (IPC)**

G01S

Le présent rapport a été établi pour toutes les revendications

| Lieu de la recherche | Date d'achèvement de la recherche | Examinateur |
|---|---|---|
| Munich | 11 avril 2025 | Zaneboni, Thomas |

EPO FORM 1503 03.82 (P04C02)

**EP 4 571 356 A1**

**ANNEXE AU RAPPORT DE RECHERCHE EUROPEENNE
RELATIF A LA DEMANDE DE BREVET EUROPEEN NO.**

EP 24 21 5535

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

11-04-2025

| Document brevet cité au rapport de recherche | Date de publication | Membre(s) de la famille de brevet(s) | Date de publication |
|---|---|---|---|
| WO 2020016250 A1 | 23-01-2020 | CN 112823283 A | 18-05-2021 |
| | | EP 3824280 A1 | 26-05-2021 |
| | | FR 3084166 A1 | 24-01-2020 |
| | | KR 20210042907 A | 20-04-2021 |
| | | US 2022003721 A1 | 06-01-2022 |
| | | WO 2020016250 A1 | 23-01-2020 |

EPO FORM P0460

Pour tout renseignement concernant cette annexe : voir Journal Officiel de l'Office européen des brevets, No.12/82

47

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2020016250 A

**Littérature non-brevet citée dans la description**

- **G. MONTALDO et al.** Cohérent plane-wave compounding for very high frame rate ultrasonography and transient elastography. *IEEE Trans. Ultrason., Ferroelect. Freq. Control*, 2009, vol. 56, 489-506
- **COUADE et al.** Ultrafast imaging of the heart using Circular Wave Synthetic Imaging with Phased Arrays. *IEEE International Ultrasonics Symposium*, 2009
- **WILLIAM LAMBERT et al.** Reflection Matrix Approach for Quantitative Imaging of ScatteringMedia. *Phys. Rev. X*, 2020, vol. 10, 021048
- **WILLIAM LAMBERT et al.** Ultrasound Matrix Imaging - Part I: The Focused Reflection Matrix, the F-Factor and the Role of Multiple Scattering. *IEEE Trans. Med. Imag.*, 2022, vol. 41, 3907-3920